# EUROPEAN PATENT SPECIFICATION

(11) **EP 4 732 014 B1**
(45) Date of publication and mention of the grant of the patent: **03.06.2026**
(21) Application number: 24790722.3
(22) Date of filing: 11.10.2024
(51) Int. Cl.: G01N 33/575, G01N 33/57505

(54) **METHODS, REAGENTS AND KITS FOR DETECTING MINIMAL/MEASURABLE DISEASE IN CHRONIC LYMPHOCYTIC LEUKEMIA (CLL)**
VERFAHREN, REAGENZIEN UND KITS ZUM NACHWEIS VON MINIMALEN/MESSBAREN ERKRANKUNGEN BEI CHRONISCHER LYMPHOZYTISCHER LEUKÄMIE (CLL)
PROCÉDÉS, RÉACTIFS ET KITS POUR DÉTECTER UNE MALADIE MINIMALE/MÉASURABLE DANS LA LEUCÉMIE LYMPHOÏDE CHRONIQUE (LLC)

(30) Priority: 12.10.2023 EP 23203269
(43) Date of publication of application: 29.04.2026
(73) Proprietor: Stichting EuroFlow, 7202 AG Zutphen (NL)
(72) Inventor: ORFAO DE MATOS CORREIA E VALE, José Alberto, 37008 Salamanca (ES); FLORES-MONTERO, Juan Alejandro, 37008 Salamanca (ES); BÖTTCHER, Sebastian, 18057 Rostock (DE); ENGELMANN, Robby, 18057 Rostock (DE)
(74) Representative: V.O.
(86) International application number: PCT/NL2024/050561
(87) International publication number: WO 2025/080137

(56) References cited:
- US-A1- 2018 140 664
- US-A1- 2021 278 409
- EDWARDS KURTIS ET AL: "The role of CD180 in hematological malignancies and inflammatory disorders", vol. 29, no. 1, 17 July 2023 (2023-07-17), XP093124807, ISSN: 1528-3658, Retrieved from the Internet <URL:https://link.springer.com/article/10.1186/s10020-023-00682-x/fulltext.html> DOI: 10.1186/s10020-023-00682-x

## Description

The invention relates to the field of leukemia/lymphoma diagnosis, more specifically to the detection of minimal numbers of leukemia/lymphoma cells in chronic lymphocytic leukemia (CLL) patients after therapy has started, as well as to the identification and monitoring of small clones of B-cells in subjects with CLL-type monoclonal B cell lymphocytosis (MBL).

In the last decades, treatment of CLL has evolved substantially with progressively higher rates of complete remission (CR) according to conventional criteria. However, many patients who achieve CR, still show relapse and disease progression, pointing out the need for more sensitive molecular and/or immunophenotypic assays for detection of leukemic CLL cells that persist at minimal levels during and after therapy. For this purpose, along the years, both molecular techniques (allele-specific oligonucleotide quantitative PCR (ASOqPCR) and next generation sequencing (NGS) of rearranged immunoglobulin genes) and flow cytometric immunophenotyping techniques have been developed to reach an MRD threshold of 10⁻⁴ in virtually every individual CLL patient. Clinical studies have demonstrated the utility of the above molecular and flow cytometric approaches for MRD detection in evaluating the quality of CR, as a powerful treatment-independent prognostic factor, and more recently also, as a biomarker for re-stratification of CLL patient at risk early, after starting therapy, and for supporting treatment decisions in individual CLL patients, particularly in the context of clinical trials. Among the MRD methods, flow cytometry is the most widely used, due to its speed, worldwide availability, and its sensitivity, similar to that of ASOqPCR and NGS. Based on the sensitivity reached by the available MRD approaches, a consensus sensitivity of at least 10⁻⁴ should be reached in the clinical settings(1). Nevertheless, virtually all patients that reach undetectable MRD at a level below 10⁻⁴, turn MRD-positive during the course of the disease. Clinical data show a direct relationship between MRD level and progression-free survival (PFS), with shorter PFS in high level (> 10⁻²) vs intermediate level (10⁻² to 10⁻⁴) vs. undetectable MRD (< 10⁻⁴ according to current definition)(2). Recent clinical studies using NGS suggest that this relationship is maintained beyond the 10⁻⁴ threshold, as patients with MRD below 10⁻⁵ had a longer PFS compared to patients with MRD levels between 10⁻⁴ and 10⁻⁵ (3-5).

The sensitivity of flow cytometric MRD assays mostly depends on the specific combination of markers used to identify the residual CLL cells and to distinguish them from all other cells coexisting in the sample, and from the total number of cells analyzed in that sample. Since 2017, EuroFlow has developed new standard operating procedures for sample preparation and cell staining for high-sensitive MRD detection by next-generation flow cytometry(6,7), which are described in detail at www.EuroFlow.org. However, so far, no unique reagent composition for high-sensitive flow cytometric MRD monitoring in CLL has been designed, which can reach below 10⁻⁵, even close to 10⁻⁶.

In the late seventies and early 80's, monoclonal antibodies specific for mature/peripheral B-lymphocytes were generated, from which CD19 and CD20 emerged as the most useful because of their high levels of expression on virtually all mature B lymphocytes. From both markers, CD19 was considered a pan-B marker as its expression already starts at the earliest stages of maturation of CD34+ B-cell precursors stages in the bone marrow, and it is maintained along all subsequent B cell maturation stages, including memory B cells and plasmablasts circulating in blood, as well as a major fraction of antibody-secreting plasma cells in BM. In contrast, CD20 expression emerges later in BM B-cell precursors and is downregulated on post-germinal center B cells during maturation towards peripheral blood plasmablasts and plasma cells. In parallel, early immunophenotypic studies in CLL B cells, revealed a unique CD19+ CD5+ immunophenotype. Although, at that time, CD5 was believed to be a T cell specific marker aberrantly expressed in CLL, subsequent studies revealed that a minor fraction of all normal B lymphocytes circulating in blood and present in both lymphoid tissues and BM (e.g., normal transitional/immature B lymphocytes), showed a similar CD19+ CD5+ phenotype to CLL cells(8,9). Despite this, subsequent studies revealed that in fact once compared to normal CD19+ CD5+ B-lymphocytes, leukemic cells from CLL patients display unique patterns of antigen expression including abnormally low levels of expression of CD20(10), CD22(11), light chain-restricted surface membrane expression of immunoglobulins (sIg) (12) and CD79b (13), combined with higher expression of CD43 (14) and CD11c (15). Based on these altered (CLL-associated) immunophenotypes, distinction between normal CD19+ CD5+ B-cells and CLL B cells could be achieved at diagnosis and even to some extent, after therapy, based on the differential expression of markers such as CD79b, CD22 and CD20 downregulated in CLL cells vs. normal CD5+ B lymphocytes (10,11,13).

In 2005, Barrena *et a*l. identified CD81 to be also abnormally downregulated in CLL (16). Based on these findings the European Research Initiative in CLL (ERIC) proposed in 2007 a first consensus 4-color antibody panel for MRD detection in CLL, which was based on combinations of pairs of informative markers to be combined with both CD19 and CD5 (17). Thus, the proposed 4-color panel included a total of five 4-color tubes consisting of three core CLL MRD tubes - 1) CD20, CD38, CD19 and CD5; 2) CD81, CD22, CD19 and CD5; and 3) CD43, CD79b, CD19 and CD5- plus a fourth back-up tube -4) sIg kappa, sIg lambda, CD19 and CD5- and, a fifth (CD45, CD14, CD19 and CD3) tube for counting the percentage of CD19 B-cells from all leucocytes and assessing the number of potential CD3+ CD19+ cell doublets from MRD counts. With this ERIC 4-color panel together with a standardized manual gating procedure for identification of CLL cells and their distinction from other normal residual B-lymphocytes, an MRD sensitivity of down to 10⁻⁴ was reached in most CLL patients. However, due to the variable immunophenotypic profile of CLL cells between individual patients, the specific value of each of the 4-color tubes varied substantially, depending on the phenotypic profile of individual cases. In subsequent years, Raponi *et al*. (18) proposed variants of these 4-color panels, using different fluorochromes for the most relevant markers, e.g., 1) CD20, CD5, CD3 and CD19; 2) CD20, CD38, CD5 and CD19; and, 3) sIg kappa, sIg Lambda, CD19 and CD5. Despite these adaptations, no increase in MRD sensitivity could be reached below 10⁻⁴. At the same time, the above reagent panels required relatively large volumes of sample and long acquisition times due to the use of multiple (e.g., three or more) 4-color antibody combinations. With the availability of ≥6-color flow cytometry instruments in diagnostic laboratories, the ERIC group proposed in 2013 a new 6-color consensus panel. Therein, the same markers used in their previous five 4-color tubes (except the CD3 marker used for positive exclusion of T-cells) were rearranged in only two 6-color tubes for greater consistency: 1) CD3, CD38, CD5, CD19, CD79b and CD20; and, 2) CD81, CD22, CD5, CD19, CD43 and CD20 (19). However, despite this more robust MRD detection approach, the sensitivity of the new panel remained at most at 10⁻⁴.

Since 2013, only a few additional markers have been identified which might be used in combination with the markers in the ERIC 6-color panel for improved detection of MRD in CLL. These include CD160 (20) and ROR1 (21), together with both CD27 and CD200 (22). As per the recommendations by Farren *et al*. in 2016 (20), CD160 is combined with CD5, CD19, CD45, CD2 and CD23 in a 6-color antibody combination, while according to DePropis *et al*. in 2020 (21) ROR1 might be combined with CD19, CD5 and CD20 in a 4-color antibody mixture.

Bento al et. (Cytometry 2020; 98:455-535) disclosed an 8-color tube with dried reagents composed of CD81FITC, ROR1PE, CD79bPC5.5, CD19PC7, CD5APC, CD43APCA750, CD20PB, and CD45KrO named DuraClone^{®} RE CLB, for the flow cytometric detection of MRD in CLL samples. The lowest MRD frequency detected was 0.01%.

The EuroFlow Consortium disclosed in WO2013/187765 and US2018/140664 the combined use of CD27 and CD200 together with a set of between 6 (CD5, CD19, CD81, CD79b, CD3 and either CD22 or ROR1), 8 (CD5, CD19, CD81, CD79b, CD3, CD22, ROR1 and CD20) and 10 (CD5, CD19, CD81, CD79b, CD3, CD22, ROR1, CD20 plus CD38 and CD43) markers selected from those previously proposed in the literature and arranged into specific 8-, 10- and 12-color antibody combinations (22). Despite the potential positive contribution of some of these markers, particularly ROR1 and CD27, the sensitivity of the proposed antibody panels remained at 10⁻⁴.

In parallel, Rawstron *et a*l. selected in 2016 the CD19, CD20, CD5, CD43, CD79b and CD81 tube as the core tube for MRD detection in CLL, to which additional markers like ROR1 and CD200 could be added into an ≥8-color CLL MRD assay with a claimed sensitivity of down to 10⁻⁶ in dilutional experiments. However, no data on real-life CLL patient series were provided to demonstrate and enable the reach of such low MRD levels in routine practice (23). Already in 2013, Sartor and Goettlieb proposed such 10-color antibody combination for MRD detection in CLL based on the core CD19, CD20, CD5, CD81, CD79b and CD43 ERIC markers to which other markers proposed by ERIC such as CD38, CD22 were added in addition to the CD3 T-cell exclusion marker and CD45 (24). However, like EuroFlow, also ERIC and others failed to demonstrate the high sensitivity of 10⁻⁶, or even 10⁻⁵, in prospective real-life series of CLL patients; in fact, flow cytometric MRD according to the ERIC consensus panel appeared to be less sensitive than NGS (23). Of note, no dilutional experiments of CLL cells in bone marrow samples were performed to further confirm the claimed higher sensitivity of the proposed assays in BM, which is a notoriously more complex type of sample for MRD studies compared to blood, due to the presence of a broader variety of immature and mature B cells, including B cell precursors and plasma cells.

In the current era of immunotherapies, new limitations for the classical MRD antibody panels are encountered. One of the first examples has been the use of CD20 as an aberrantly low expressed marker in CLL. Already early in the CLL treatment protocols with anti-CD20 antibodies such as Rituximab, it appeared that CD20 expression is downregulated in CLL MRD cells as well as in normal BM and blood B cells. Likewise, also CD200 expression (as an aberrant CLL marker) is downregulated upon treatment with novel CLL-directed therapies such as the BTK-inhibitor Ibrutinib. Obviously, this hampers the usage of CD200 in MRD panels.

In parallel to the above CLL studies, the presence of small clones of CLL-like B cells (<500 cells/microliter) present in blood of otherwise healthy individuals has been reported across different populations and termed CLL-like monoclonal B lymphocytosis (MBL) (25). Immunophenotypically, MBL cells from most of these individuals display a CLL-like immunophenotype which fully overlaps with that of leukemic cells from CLL patients. Consequently, identical flow cytometry immunophenotyping strategies and reagent combinations as used for MRD detection in CLL have been applied for MBL detection and monitoring. In addition, some extra markers have been included for MBL monitoring, based on 2-color to 8-color panels, but these extra markers did not prove critical for detection of small numbers of circulating CLL-like MBL, such as CD23 (26), CD11a, IgM, IgG and CD10 (27), FMC7 (28), HLADR, IgA, IgD (29), bcl2 (30), CD185 and CD305 (31).

More recently, the EuroFlow Lymphocyte Screening Tube (LST) which combines 12 antibodies with only 8 different fluorochrome labels (CD3, CD45, CD 19+TCRgamma-delta, sIg Kappa+CD56, sIg Lambda+CD8, CD4+CD20, CD5 and CD38) has proven particularly useful because of reaching sensitivities of between 10⁻⁵ and 10⁻⁶ in blood of MBL subjects (32). Of note, detection of CLL-like MBL clones with LST reagent strongly relies on the expression of CD5 with dim CD20 on the clonal MBL cells. Because of this, the more heterogeneous expression of CD5 and CD20 in CLL compared to MBL, and the frequent use of anti-CD20 therapy in CLL, the LST did not prove useful for MRD detection in CLL. Moreover, the sensitivity of the LST antibody combination, as well as other antibody panels previously proposed for the detection of CLL or CLL-like cells in blood, decreases substantially in bone marrow samples compared to blood samples.

As explained above, the currently available "classical" flow cytometric MRD approaches in CLL have important limitations and pitfalls. These limitations are at least related to:
1. The high phenotypic heterogeneity of CLL cells between patients and within patients;
2. The increasing usage of immunotherapies targeting cell-surface proteins (e.g., anti-CD20) and/or signaling molecules (e.g., BTK-directed therapies) that might modulate the cell surface membrane expression of classical CLL MRD markers;
3. The higher sensitivity required for MRD detection both in blood and BM of CLL patients, because of a need for better prognostification in patients who achieve MRD ≤10⁻⁴. With increasing efficacy of the treatment, an increasing proportion of patients achieve MRD levels ≤10⁻⁴.

US 2018/140664 discloses a panel of biomarkers for detecting MRD in CLL comprising: CD38, CD81, CD19, CD27 and CD5.

The present inventors aimed at providing improved reagent compositions that overcome at least part of these limitations and pitfalls. In particular, they sought to develop novel antibody panels, methods and kits that allow for improved staining of e.g. blood and bone marrow (BM) samples from patients diagnosed with CLL and from MBL subjects, reaching sensitivities of detection of minimal/measurable disease of at least 10⁻⁵, even down to 10⁻⁶. Preferably, the novel approach works independently from CLL-targeted therapies and is fully operator-independent, i.e. not requiring manual gating of CLL.

To that end, they performed extensive testing and searching for more robust MRD antibody combinations which work simultaneously in blood and BM samples from both CLL patients and MBL subjects.

It was surprisingly found that these goals can be met by staining a sample for a set of six core markers CD180, CD38, CD81, CD19, CD27 and CD5. This approach allows for the identification of mature CD19+ B-lymphocytes and, within them, of their major CD5- B-lymphocyte and minor CD5+ B-lymphocyte compartments. In particular, by including CD180 in the set of core markers it is possible to discriminate between normal/reactive CD5+ CD27-/+ CD180^{hi} B-lymphocytes and CD5+ CD27+ CD180^{-/low} CLL or CLL-like MBL cells in the later CD19+ CD5+ minor B-lymphocyte compartment. This set of core markers, optionally supplemented with further markers, can be used to monitor minimal/measurable residual disease (MRD) during and after therapy with chemotherapeutic agents, immunotherapy or combinations thereof.

Accordingly, the invention provides a reagent composition for the cytometric detection of minimal residual disease (MRD) in chronic lymphocytic leukemia (CLL), the reagent composition comprising a panel (i.e. admixture) of at least six antibodies conjugated to a detectable label, the panel comprising at least antibodies directed against the markers CD180, CD38, CD81, CD19, CD27 and CD5.

A panel of antibodies against these six core markers is not disclosed or suggested in the art. Whereas US2018/140664 A1 discloses an MRD-CLL cocktail of 12 antibodies against several markers including CD38, CD81, CD19, CD27 and CD5, it is silent about using CD180 as additional marker. Furthermore, the cocktail also contains antibodies against cell surface markers that are often targeted and/or downmodulated by anti-CLL treatment (CD20 and CD200).

A role of CD180 in hematological malignancies was reviewed by Kurtis et al. (Molecular Medicine (2023, vol. 29, no.1), suggesting that CD180 could be utilized to provide better resolution in CLL disease prediction or therapy response. Importantly however, this bears no relevance for detecting MRD in CLL, let alone that it implies that CD180 is advantageously used to improve detection sensitivity in MRD-CLL. Hence, a skilled person had no reason to believe or expect that staining for CD180 would significantly improve the sensitivity of detection of minimal/measurable disease in CLL.

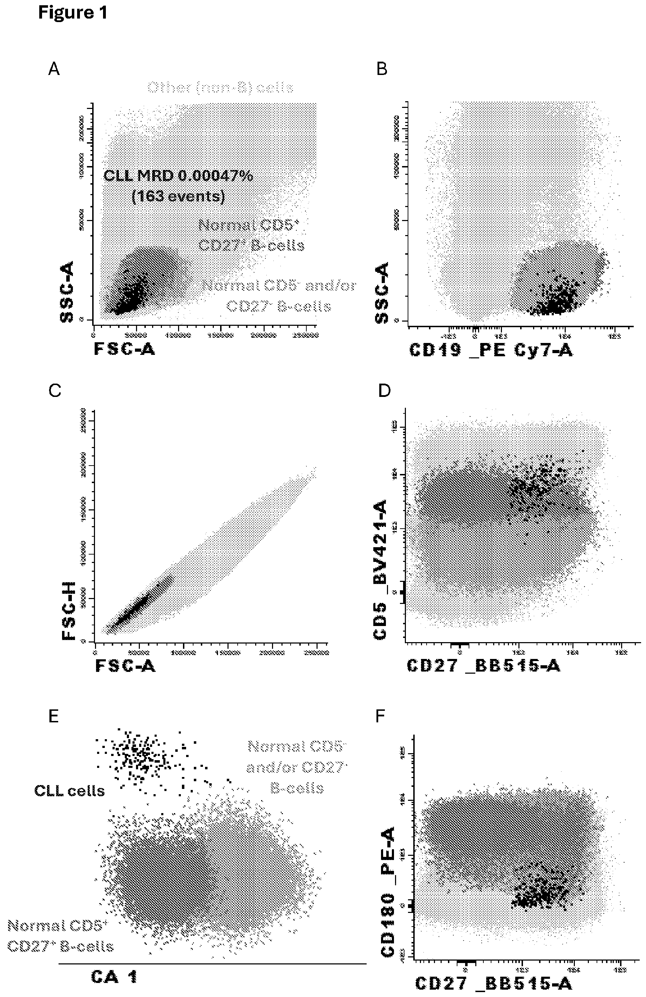

According to the invention, at least the antibodies against the markers CD5, CD19, CD27 and CD180 are each conjugated to a distinct detectable label. In one aspect, all six core markers are conjugated to a distinct detectable label. In another aspect, the antibodies against core markers CD38 and CD81 may be conjugated to the same detectable label such that within one reagent composition only 5 distinct (distinguishable) labels are required. Hence, in a preferred embodiment of the invention, the antibody reagents against the CD38 and CD81 markers are both conjugated to the same "first" label (thus forming a CD38/CD81 "pair") whereas the remaining CD5, CD19, CD27 and CD180 antibodies are conjugated each, with a different second, third, fourth and fifth label, respectively (Table 1A and Figure 1A).

An antibody panel comprising antibodies against the 6 core markers according to the present invention are advantageously supplemented with one or more antibodies against further selected markers that improve the application window and/or detection sensitivity of MRD in CLL. For economical and/or practical reasons, e.g. to allow discrimination between distinctly labeled antibodies using conventional (clinical) flow cytometric equipment, it is preferred that the antibody panel contains up to 20, or up to 19, or up to 18, or up to 17, or up to 16 distinctly (uniquely) labeled antibodies. Exemplary detection reagents include 16-label, 15-label, 14-label, 13-label, 12-label, 11-label, 10-label, 9-label, 8-label, 7-label and 6-label reagent compositions.

Additionally, it may be preferred that the selected further markers do not comprise cell surface markers that are often targeted by anti-CLL treatment, such as CD20 and CD200. Accordingly, in one embodiment the MRD-CLL detection reagent comprises a panel of conjugated antibodies directed against the markers CD180, CD38, CD81, CD19, CD27 and CD5, and wherein the reagent does not comprise cell surface marker(s) whose expression level may be subject to change e.g. downregulation, due to anti-CLL treatment. In a specific aspect, the MRD-CLL detection reagent does not contain antibodies against CD20 and/or CD200.

Other markers that may be excluded, e.g. for reasons that they do show no staining or no difference between CLL versus mature B cells, include CD74, CD84, CD160, IgM/D, and CD23 and CD22.

In one embodiment, the set of core markers is supplemented with at least two, preferably at least three, more preferably at least four, most preferably at least five additional markers. Suitable further markers include markers that are aberrantly expressed by both CLL as well as MBL cells when compared to normal immature, to naive, to memory B lymphocytes and/or to plasmablasts and plasma cells. For example, provided is an antibody panel comprising antibodies against the 6 core markers, supplemented with one or more antibodies against further markers selected from the group consisting of CD43, CD79b, ROR1, human immunoglobulin (Ig) kappa (κ) and Ig lambda (λ) light chains, CD45, CD3, preferably wherein each further antibody within the set is conjugated to a distinct label.

In some aspects, the core markers may be supplemented with one or more of the markers CD43, CD79b and ROR1. Therefore, in one embodiment the invention provides a reagent composition comprising a panel of antibodies conjugated to a detectable label, the panel comprising antibodies directed against the markers CD180, CD38, CD81, CD19, CD27 and CD5, and further comprising conjugated antibodie(s) directed against one or more of the markers CD43, CD79b and ROR1. In a specific aspect, the reagent composition comprises conjugated antibodie(s) directed against the markers CD43, CD79b and ROR1.

Still further, the reagent composition may further comprise a set of conjugated antibodies directed against the markers human immunoglobulin (Ig) kappa (κ) and Ig lambda (λ) light chains, wherein each antibody within the set is conjugated to a distinct label. Ig kappa and Ig lambda light chains can be detected in plasma cells using intracellular staining protocols known in the art.

Good results can be obtained with a reagent composition further comprising a conjugated antibody directed against CD45 and/or a conjugated antibodies directed against the marker CD3. In a preferred embodiment, the reagent composition comprises a distinctly conjugated antibodies directed against CD45 and CD3.

According to the invention, the antibodies may be conjugated to any type of label that can be detected by cytometry. In one embodiment, the detectable label is a fluorochrome. Alternatively, the antibodies are suitably conjugated to a metal isotope.

Exemplary reagent compositions may comprise or consist of an admixture of 6, 7, 8, 9, 10 or 11 conjugated antibodies. Preferred reagent compositions comprise or consist of an admixture of 12, 13 or 14 conjugated antibodies.

For example, provided is a 6- to 9-label reagent composition comprising or consisting of 7 to 8 conjugated antibodies.

In one aspect, the invention provides a 6- or 7- label (color) reagent composition consisting of 7 antibodies against the markers:
(i) CD19, CD5, CD38, CD81, CD27, CD180 and CD43;
(ii) CD19, CD5, CD38, CD81, CD27, CD180 and CD79b; or
(iii) CD19, CD5, CD38, CD81, CD27, CD180 and ROR1.

In one aspect, the invention provides a 7- or 8- label (color) reagent composition consisting of 8 antibodies against the markers:
(iv) CD19, CD5, CD38, CD81, CD27, CD180, CD43 and CD79b;
(v) CD19, CD5, CD38, CD81, CD27, CD180, CD79b and ROR1; or
(vi) CD19, CD5, CD38, CD81, CD27, CD180, ROR1 and CD43.

In one aspect, the invention provides a 8- or 9- label (color) reagent composition consisting of 9 antibodies against the markers:
(vii) CD19, CD5, CD38, CD81, CD27, CD180, CD43, CD79b and ROR1.

In another example, the invention provides an 8- to 11-label reagent composition comprising or consisting of 9 to 11 conjugated antibodies.

In one aspect, the invention provides an 8- or 9- label (color) reagent composition consisting of 9 antibodies against the markers
(viii) CD19, CD5, CD38, CD81, CD27, CD180, CD43, IgKappa and IgLambda;
(ix) CD19, CD5, CD38, CD81, CD27, CD180, CD79b, IgKappa and IgLambda; or
(x) CD19, CD5, CD38, CD81, CD27, CD180, ROR1, IgKappa and IgLambda.

In one aspect, the invention provides a 9- or 10- label (color) reagent composition consisting of 10 antibodies against the markers
(xi) CD19, CD5, CD38, CD81, CD27, CD180, CD43, CD79b, IgKappa and IgLambda;
(xii) CD19, CD5, CD38, CD81, CD27, CD180, CD79b, ROR1, IgKappa and IgLambda; or
(xiii) CD19, CD5, CD38, CD81, CD27, CD180, ROR1, CD43, IgKappa and IgLambda.

In one aspect, the invention provides a 10- or 11- label (color) reagent composition consisting of 11 antibodies against the markers
(xiv) CD19, CD5, CD38, CD81, CD27, CD180, CD43, CD79b, IgKappa and IgLambda.

In one aspect, the invention provides a 11- or 12- label (color) reagent composition consisting of 12 antibodies against the markers
(xv) CD19, CD5, CD38, CD81, CD27, CD180, CD43, CD79b, ROR1, IgKappa, IgLambda and CD45.

In one aspect, the invention provides a 12- or 13- label (color) reagent composition consisting of 13 antibodies against the markers
(xvi) CD19, CD5, CD38, CD81, CD27, CD180, CD43, CD79b, ROR1, IgKappa, IgLambda, CD45 and CD3.

In a specific embodiment, the invention provides a reagent composition as depicted in any one of Tables 1-5 herein below. In one aspect, the reagent composition is defined in Table 1. In another aspect, the reagent composition is defined in panel 2A, 2B or 2C of Table 2. In yet another aspect, the reagent composition is defined in panel 3A, 3B or 3C of Table 3. In a still further aspect, the reagent composition is defined in panel 4A or 4B of Table 4. Also provided is reagent composition as defined in panel 5A, 5B or 5C of Table 5.

A further aspect relates to a diagnostic kit, comprising at least one reagent composition according to the invention, optionally together with instructions for use, buffer, and/or control samples. Such kit is particularly suitable for cytometric detection of minimal residual disease (MRD) in in chronic lymphocytic leukemia (CLL), more specifically to the detection of minimal numbers of leukemia/lymphoma and MBL (i.e., clonal B cells) in blood and in bone marrow (BM) of patients diagnosed CLL and of subjects presenting with monoclonal B-cell lymphocytosis (MBL), respectively.

In a further embodiment, the invention provides a cytometric method for detecting MRD in CLL and/or for more specifically to the detection of minimal numbers of leukemia/lymphoma and MBL (i.e., clonal B cells) in blood and in bone marrow (BM) of patients diagnosed CLL and of subjects presenting with MBL. The method comprises the steps of:
(i) contacting one or more aliquots of a biological sample comprising leukocytes obtained from a subject with a reagent composition according to the invention;
(ii) analyzing leukocytes in said aliquot(s) in a (flow or mass) cytometer; and
(iii) gating on cells for expression of the selected markers detected by the antibodies present in the reagent composition; and
(iv) distinguishing between normal and malignant cells, based on the expression profile of the multiple markers.

The method may involve multivariate analysis, preferably principal component analysis (PCA) and/or canonical analysis.

Also provided is the *in vitro* use of a reagent composition, diagnostic kit and/or or method according to the invention in detecting MRD in CLL and/or for more specifically to the detection of minimal numbers of leukemia/lymphoma and MBL (i.e., clonal B cells) in blood and in bone marrow of CLL patients and of subjects presenting with MBL.

See Table 6 herein below for the representative expression of the CLL-MRD panel markers on normal B cells and CLL/MBL cells. Marker expression levels were determined arbitrarily based on the median fluorescence intensity (MFI) measure and arbitrarily classified as negative (-) clearly positive (+), bright (++) and very bright (+++).

### LEGEND TO THE FIGURES

Figure 1: Illustrating example of the identification of normal mature CD5⁺ and CD5- populations of B lymphocytes (dark gray and medium gray events, respectively) and their clear-cut discrimination from residual CLL tumor cells (black dots) using the 5-label reagent composition tube 1 of Table 1A. In light grey all other normal populations are shown in all plots except the one in the lower right corner.

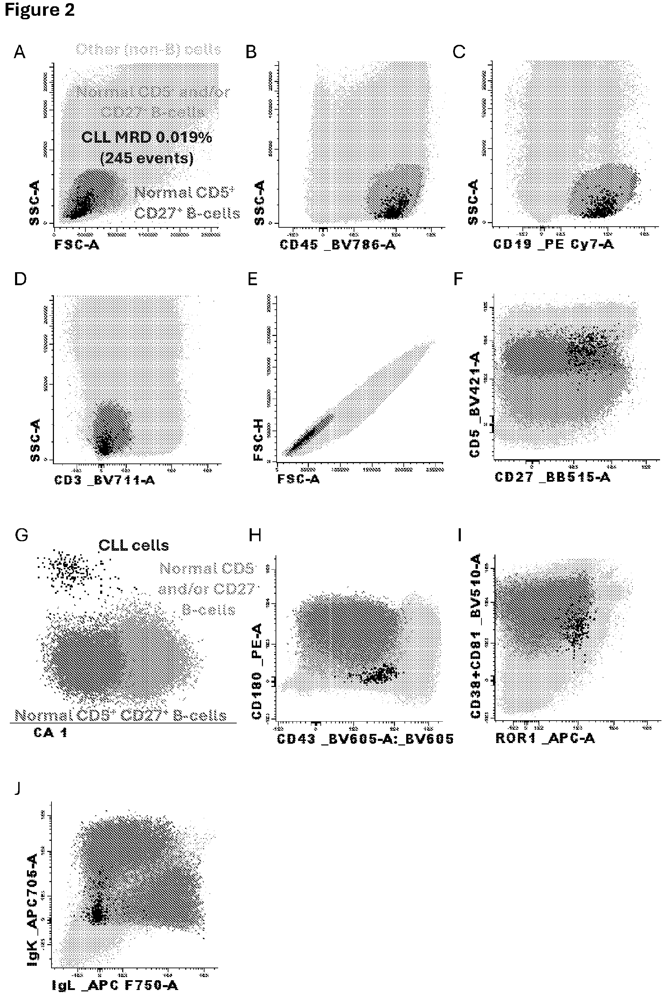

Figure 2: Illustrating example of the distinct immunophenotypic profile of normal (CD5- and reactive CD5⁺, medium and dark grey dots, respectively) B lymphocytes vs. aberrant CLL tumor cells (black dots) using the unique 12-label, 13-antibody reagent composition according to the upper panel of Table 5B.

### DETAILED DESCRIPTION

The invention relates in some embodiments to improved and advantageous reagent compositions and kits comprising the same. The term "reagent composition" (or "cytometric panel") as used herein relates to a cocktail

(admixture) of antibodies (or other specific antigen-binding agents) which are conjugated to (directly or indirectly) a detectable label.

Antibodies, or immunoglobulins, comprise two heavy chains linked together by disulfide bonds and two light chains, each light chain being linked to a respective heavy chain by disulfide bonds in a "Y" shaped configuration. The variable domains of each pair of light and heavy chains form the antigen binding site. The isotype of the heavy chain (gamma, alpha, delta, epsilon or mu) determines immunoglobulin class (IgG, IgA, IgD, IgE or IgM, respectively). It should be understood that when the terms "antibody" or "antibodies" are used, this is intended to include intact antibodies, such as polyclonal antibodies or monoclonal antibodies (mAbs), as well as proteolytic fragments thereof such as the Fab or F(ab')2 fragments. Further included within the scope of the invention are chimeric antibodies; recombinant and engineered antibodies, and fragments thereof, as well as other molecules comprising at least an antigen binding site (retaining the antigen binding capacity) of an antibody. In a preferred embodiment, the composition comprises a panel of conjugated monoclonal antibodies. (Monoclonal) antibodies against the indicated markers can be commercially obtained from various companies, including Becton/Dickinson (BD) Biosciences, Biolegend, Dako, Beckman Coulter, CYTOGNOS, Caltag, Myltenyi, Pharmingen, Exbio, Sanquin, Invitrogen, and the like.

Antibodies for use in the present invention are conjugated to a label that is detectable by cytometry, e.g. by flow or mass cytometry. Suitable types of detectable labels include fluorochromes (fluorophores), quantum dots, and metal-isotope labels. Detectable labels can be covalently conjugated to antibodies using methods known in the art, e.g. through reactions with thiol or amine groups. Typically, fluorochromes containing isothiocyanate, succinimidyl ester, or sulfonyl chloride reactive groups are conjugated to amines on the antibody molecules. Exemplary protocols for conjugating antibodies with heavy-metal isotopes are disclosed by Han et al., Nat Protoc . 2018 Oct;13(10):2121-2148.

As will be understood, to facilitate simultaneous detection, all antibodies present in a reagent composition of the invention are conjugated to the same type of detectable label, e.g. all are fluorochrome conjugated or all are isotope labeled. In addition, flow cytometry uses the light properties scattered from cells or particles for identification or quantitative measurement of physical properties.

In one embodiment, the antibody is conjugated to a fluorochrome. A fluorochrome (or "fluorophore") is a chemical which can absorb energy from an excitation source (laser beam) and emit photons at a longer wavelength (fluorescence), which is captured by optical detectors of the flow cytometer. Suitable fluorochromes for conjugating antibodies are known in the art. Each fluorophore has a characteristic peak excitation and emission wavelength, and the emission spectra often overlap. Consequently, the combination of labels which can be used depends on the wavelength of the lamp(s) or laser(s) used to excite the fluorochromes and on the detectors available. The fluorochromes are preferably selected for brightness, limited spectral overlap and limited need for compensation, stability, etc.

A wide range of fluorochromes can be used as labels in flow cytometry. Fluorochromes of particular use in a reagent composition according to the invention include those of the Brilliant Violet (BV) series, such as BV421 or functional equivalent thereof (e.g., Pacific Blue, HV450, SuperNovaV428 or VioBlue), BV510 or functional equivalent thereof (e.g., HV500, OC515, Pacific Orange, StarBrightV515 or SparkViolet438), BV605 or functional equivalent thereof (e.g., SuperBright600 or StarBriaghtV610), BV650 or functional equivalent thereof (e.g., StarBrightV670), BV711 or functional equivalentthereof (e.g., SuperBright700 or StarBrightV710), BV786 or functional equivalent thereof (e.g., BV785, StarBrightV790 or SuperBright780), fluorescein isothiocyanate (FITC) or functional equivalent thereof (e.g. BB515, VioBrightFITC, VioBright515, CFluorB515 or AlexaFluor488), PerCP Cy5.5 or functional equivalent thereof (e.g., BB700, RealBlue705, StarBrightB700 or PerCPVio700), phycoerythrin (PE) or functional equivalent thereof (e.g., StarBrightYG570, RealYellow586, SparkYG581 or CFluorYG575), phycoerythrin/CF594 (PE CF594) or functional equivalent thereof (e.g., PEDazzle594, PEVio610 or RealYellow610), phycoerythrin/cyanine5 (PE-Cy5) or functional equivalent thereof (e.g., PE-Fire700 or RealYellow703), phycoerythrin/cyanine7 (PE-Cy7) or functional equivalent thereof (e.g., PE-Vio770, RealYellow775 or RealBlue780), allophycocyanine (APC) or functional equivalent thereof (e.g., Alexa647, DY634 or StarBrightR670), AF700 (AlexaFluor700) or functional equivalent thereof (e.g., R718, APCR700, SparkRed710, StarBrightR712 or APC705), and allophycocyanine/H7 (APC-H7) or functional equivalent thereof (e.g. APC-Cy7, APC-Alexa750, APCVio770, APCC750 or APC-Fire750), .

The following panel of fluorochromes is of particular use in a reagent composition according to the invention: (L1) PECy7 or functional equivalent thereof; (L2) BV421 or functional equivalent thereof; (L3) BV510 or functional equivalent thereof; (L4) BB515 or functional equivalent thereof; (L5) BV650 or functional equivalent thereof; (L6) phycoerythrin (PE) or functional equivalent thereof; (L7)BV605 or functional equivalent thereof ; (L8) PerCPCy5.5 or functional equivalent thereof; (L9) allophycocyanine (APC) or functional equivalent thereof (e.g. Alexa647); (L10) APC705 or functional equivalent thereof; (L11) APC-Fire750 or functional equivalent thereof (L12) BV786 or functional equivalent thereof; (L13) BV711 or functional equivalent thereof.

For example, in the stainings performed with the 12-color CLL reagent set as herein disclosed, the following Mab reagents were used: CD19 (J3.119) - phycoerythrin-cyanine 7 (PECy7) (Beckmann Coulter); CD5 (L17F12) - Brilliant Violet 421 (BV421) (Biolegend); CD38 (HIT2) - Brilliant Violet 510 (BV510) (Biolegend); CD81 (JS81) - BV510 (Biolegend); CD27 (M-T271) - Brilliant Blue 515 (BB515) (Becton Dickinson Biosciences, BD); CD180 - phycoerythrin (PE) (Biolegend); CD43 (1G10) - Brilliant Violet 605 (BV605) (BD); CD79b (SN8) - peridinin-chlorophyll protein-Cyanine5.5 (PerCP-Cy5.5) (BD); ROR1 (2A2) - allophycocyanine (APC) (Biolegend); IgKappa (IgK) - allophycocyanine 705 (APC705) (Cytognos); IgLambda (IgL) - allophycocyanine-Fire 750 (APC-Fire750) (Biolegend); CD45 (HI30) - Brilliant Violet 786 (BV786) (Biolegend); CD3 (SK7) - Brilliant Violet 711 (BV711) (Biolegend).

In another embodiment, the antibody is conjugated to a quantum dot. Quantum dots are sometimes used in place of traditional fluorophores because of their narrower emission peaks.

In yet another embodiment, the antibody is conjugated to a metal isotope, allowing for detection by mass cytometry, also called cytometry by time-of-flight (CyTOF). Advances in single-cell mass cytometry have increasingly improved highly multidimensional characterization of immune cell heterogeneity. The immunoassay multiplexing capacity relies on monoclonal antibodies labeled with stable heavy-metal isotopes.

Mass cytometry overcomes the fluorescent labeling limit by utilizing lanthanide isotopes attached to antibodies. This method could theoretically allow the use of 40 to 60 distinguishable labels or more. Mass cytometry is fundamentally different from flow cytometry: cells are introduced into a plasma, ionized, and associated isotopes are quantified via time-of-flight mass spectrometry. Although this method permits the use of a large number of labels, it currently has lower throughput capacity than flow cytometry. It also destroys the analysed cells, precluding their recovery by sorting. Finally, a major fraction of cells is lost before reaching the cone for measurement.

To date, a variety of rare-earth elements and noble and post-transition metal isotopes have been used in mass cytometry. For example, Han et al. (2018, Nature Protocols volume 13, pages 2121-2148) disclose protocols for conjugating monoclonal IgG antibodies with 48 high-purity heavy-metal isotopes: (i) 38 isotopes of lanthanides, 2 isotopes of indium, and 1 isotope of yttrium; (ii) 6 isotopes of palladium; and (iii) 1 isotope of bismuth.

In one embodiment, a reagent composition of the invention comprises a panel of antibodies that are conjugated to metals of different mass, in particular isotopes of metals, preferably isotopes of metals selected from the group consisting of Pr, Bi, Y, Nd, Sm, Eu, Gd, Tb, Dy, Ho, Er, Tm, Yb, Lu, Rh, La, Cd, In, Ir, Au, Pt and Pd. Specific examples include 89Y, 103Rh, 106Cd, 110Cd, 111Cd, 112Cd, 113Cd, 114Cd, 115In,116Cd, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho,166Er, 167Er, 168Er, 169Tm, 170Er, 171Yb, 172Yb, 173Yb, 174Yb, 175Lu, 176Yb, 191Ir, 193Ir, 194Pt, 197Au, 198Pt and 209Bi.

In a typical process of sample preparation for mass cytometric analysis of cells, cells are incubated (or "stained") with a panel of metal-conjugated antibodies that target antigens (markers) of interest. A DNA intercalator can be incorporated into the panel to allow determination of nucleated cells from nonnucleated cells. Cells are stained under resting or stimulating conditions and are fixed prior to analysis. The samples are typically washed to remove unbound antibody and salts and diluted to an appropriate cell concentration. Cells may then be passed in a single-cell suspension into a nebulizer, which aerosolizes the cells into droplets for introduction into the mass cytometer. Upon entering the instrument, cells travel through an argon plasma at 7000°K which completely vaporizes and ionizes the cell and the attached antibodies into a cloud of single-atom ions. The size of the cloud is largely driven by gas expansion kinetics and is relatively independent of the cell size. The ion cloud is filtered by a quadrupole to remove common biological elements with a mass less than ~ 75 Da, to leave only the heavy metal ions that were attached to the staining antibodies directed against the marker set of interest. The ions within the cloud are separated by their mass-to-charge ratio in a time-of-flight (TOF) mass spectrometer. Ion signals are integrated on a per-cell basis, resulting in single-cell measurements for analysis.

The panel of antibodies in a reagent composition provided herein may be used in some embodiments to simultaneously label a cell-containing biological sample (in suspension). In other words, the sample is typically and advantageously incubated with an entire cytometric panel as disclosed herein, thus allowing characterization of multiple cell populations in a single measurement step, using multi-parametric analysis ("multiplexing") of the antigen co-expression pattern on single cells from the sample. Accordingly, antibodies that are directed to distinct cellular targets ("markers") for which separation is desired within a panel, are labeled with distinct (non-equivalent) detectable labels, and are referred to herein as distinct conjugated antibodies. The marker is typically a (human) protein that is expressed on a cell's surface (cell surface marker). "CD" stands for cluster designation and is a nomenclature for the identification of specific (human) cell surface antigens defined by monoclonal antibodies.

In some embodiments, a reagent composition comprises antibodies against different markers, but wherein a subset, e.g. two or three, of these antibodies are conjugated to the same detectable label. See for example tube 1 of Table 1A, comprising antibodies against CD38 and CD81, each conjugated to the same detectable label. The reagent composition may comprise two or more of such pairs of antibodies, wherein the antibody within either one of the pairs is conjugated to the same detectable label, but wherein between different pairs the labels are distinguishable.

The term "kit" as used herein relates to an article of manufacture comprising at least one MRD-CLL detection reagent composition of the invention, and optionally additional reagents, e.g. reagents for cell dissociation, purification, permeabilization, control samples or antibodies, or other reagents for use in flow cytometry. The kit may further contain instructions for using the at least one reagent composition in the methods of the invention, e.g. instructions for analyzing the results measured using multi-parametric analysis as detailed herein. In one embodiment, the invention provides a diagnostic kit comprising at least one 12-color or 13-color reagent composition herein disclosed.

A cytometric method for monitoring the immune status and/or the effect of an immune modulatory treatment of a subject typically comprises the steps of: (a) contacting an aliquot of a biological sample comprising leukocytes obtained from the subject with a reagent composition as herein disclosed; (b) analyzing leukocytes in said aliquot in, depending on the type of detectable label, a flow or mass cytometer; and (c) storing and evaluating the data obtained.

For robust, reliable and reproducible flow cytometry-based diagnostics, EuroFlow Consortium has developed new standardized sample preparation methods and standard operating procedures (SOPs). These methods and SOPs allow for high-sensitive detection of MRD by next-generation flow cytometry [7], which are available and described in detail at www.EuroFlow.org.

Any type of sample known or suspected to contain leukocytes may be used directly, or after lysing non-nucleated red cells, or after density centrifugation, or after cell sorting procedures. For example, the sample is peripheral blood, bone marrow, tissue sample such as lymph nodes, adenoid, spleen, or liver, or other type of body fluid such as cerebrospinal fluid, vitreous fluid, synovial fluid, pleural effusions or ascites. Peripheral blood (PB) or bone marrow (BM) is preferred.

Preferably, step (c) of a method provided herein comprises combining the immunophenotypic information of two or more selected cell populations from multiple tubes according to the so-called nearest neighbor calculations in which individual cells from one aliquot of a sample are matched with corresponding individual cells from another aliquot of the same sample, according to their markers and scatter profile. Advantageously, the method comprises the use of software for data integration and multidimensional analysis of flow cytometry files, preferably wherein said software is INFINICYT^{™}.

In the following sections, specific preferred embodiments of the invention are presented. Whereas the embodiments shown relate to antibodies that are conjugated to a fluorochrome as detectable label, it will be understood and appreciated that variant embodiments involving other types of detectable labels, e.g. metal-isotopes, are also encompassed. As used herein, the expression "marker combination" discussed in relation to a reagent composition implies that the composition comprises conjugated antibodies directed at each marker of said marker combination.

For all flow cytometric immunophenotyping MRD studies, fresh samples can be used, such as fresh blood, bone marrow (BM), cerebrospinal fluid, saliva, tears, pleural effusions, peritoneal fluid, bronchoalveolar lavage, thymus, lymph nodes, mediastinal and abdominal masses, and spleen specimens. These samples are suitably stained and processed within 24h after collection. For blood and BM samples, the NH₄Cl-based EuroFlow bulk-lysis standard operating procedure (SOP) may be used to lyse non-nucleated red cells prior to staining (www.EuroFlow.org). Thymus, lymph nodes, mediastinal and abdominal masses, and spleen samples can be mechanically dissociated into single cell suspensions, prior to staining. Thereafter, at least 5x10⁶ leukocytes per sample aliquot may be stained with the appropriate set of monoclonal antibody (Mab) reagents.

Acquisition of the stained samples is preferably performed immediately after immunostaining, in a FACS Lyric flow cytometer (BD), using the FACSDiva^{™} software (BD). For instrument setup, calibration and monitoring, the EuroFlow instrument setup & compensation protocol for 14-color measurements may be used [30,31]. For data analysis, the Infinicyt^{™} software (Cytognos S.L.) can be employed. For each blood sample, relative and absolute (double-platform) cell counts are calculated and recorded for both the major cell population of interest and each specific cell subset. In BM, lymph node, thymus and spleen samples, only relative cell numbers may be derived.

In a first embodiment of the invention, a sample is stained with a reagent composition comprising an antibody panel directed against the markers CD180, CD38, CD81, CD19, CD27 and CD5 antibodies. This novel panel allows for the identification of mature CD19+ B-lymphocytes and within them, of their major CD5- B-lymphocyte and minor CD5+ B-lymphocyte compartments. In the later CD19+ CD5+ B-lymphocyte compartment, the novel set of markers further allows the discrimination between normal/reactive CD5+ CD27-/+ CD180^{hi} B-lymphocytes and CD5+ CD27+ CD 180^{-/low} CLL or CLL-like MBL cells (Table 1, Figure 1). The antibodies may be conjugated to different detectable labels.

In a preferred embodiment, the antibodies against the CD38 and CD81 markers are conjugated to the same detectable label (e.g. fluorochrome or metal isotope) while the antibodies against CD5, CD19, CD27 and CD180 are conjugated each, with a different second, third, fourth and fifth labels, respectively (Table 1A and Figure 1). Based on their CD38^{-/low} and CD81^{-/low} phenotype, CLL or CLL-like MBL cells can be further discriminated from normal CD38^{+/hi} and/or CD81^{hi} B cell precursors and plasma cells and from CD5+ CD38+ CD81+ immature/transitional B-cells which are more represented in BM compared to blood of both CLL patients and healthy subjects with or without CLL-like MBL (Table 1A and multivariate canonical analysis (CA1) in Figure1 lower right panel). Alternatively, antibodies directed against CD38 and CD81 are each conjugated to a different label (Table 1B).

Then, ≥10⁷ cellular events of the stained sample are measured in a flow cytometer, and flow cytometry data analyzed using conventional manual gating or automated gating procedures, based on commercially available flow cytometry data analysis software programs.

**Table 1. Illustrating example of a 5-or 6-label reagent compositions for detection of MRD and MBL in CLL and healthy subjects, respectively.**

| Panel 1A. Reagent Composition with CD38 and CD81 antibodies conjugated to the same label | | | | | | |
|---|---|---|---|---|---|---|
| Tube Version | Label (L) position | | | | | |
| | L1 | L2 | L3 | L4 | L5 | L6 |
| 1 | CD19 | CD5 | CD38 and CD81 | CD27 | - | CD180 |

| Panel 1B. Reagent Composition with CD38 and CD81 antibodies conjugated to different labels | | | | | | |
|---|---|---|---|---|---|---|
| 2 | CD19 | CD5 | CD38 | CD27 | CD81 | CD180 |

For a broader applicability and greater sensitivity, the markers CD180, CD81, CD38, CD19, CD27 and CD5 are used in combination with an additional set of between 1 and 3 markers that are aberrantly expressed by both CLL as well as MBL cells (CD43+, CD79b^{lo} and ROR1+) when compared to normal (CD19+ CD5+ CD27- CD43- CD79+ ROR1-/lo) immature, to (CD19+ CD5+ CD27- CD43-CD79+ ROR1-/lo) naïve, to (CD19+ CD5- CD27-/+ CD43- CD79+ ROR1-/lo) memory B lymphocytes and/or to (CD19-/+ CD5- CD27+ CD43-/+ CD79-/lo ROR1-) plasmablasts and plasma cells, for more clear discrimination between both CLL and CLL-like MBL cells and normal B lymphocytes and plasma cells based on 6 to 9 color antibody combinations (Table 2A to 2C).

For a broader applicability and greater sensitivity, each of the previously defined 6-color to 9-color antibody combinations may be combined with simultaneous staining with antibodies directed against human immunoglobulin (Ig) kappa and Ig Lambda light chains to confirm the restricted usage of Ig light chains or low level expression by the CLL MRD cells (similar to that observed at diagnosis in individual CLL patients, and/or the CLL-like MBL cells from healthy subjects with MBL (Table 3).

Still further, a CD45 antibody conjugated to a different unique label may be added to any of the above said antibody combinations. This allows for a more accurate identification and enumeration of CLL and MBL cells via correct identification of all (CD45⁺) leucocytes in the sample and their discrimination from cell debris (Table 4A).

In a further embodiment, an antibody against the T-cell specific CD3 cell surface protein conjugated to a unique single label, different from those used in conjugation with the markers included in the previous panels listed in Tables 1 to 3 is added for specific exclusion of T-cells, including the identification (for exclusion) of all T-B cell doublets (Table 4 and Figure 2).

Table 5 depicts exemplary CLL MRD / MBL reagent compositions comprising antibodies conjugated to distinct fluorochromes (panels A and B) or isotopes (panels C and D).

**Table 2. Illustrating example of the 6-label to 9-label reagent composition consisting of the core reagent combinations to which antibodies against the CD43, CD79b and/or ROR1 markers were added for detection of MRD in treated CLL patients and MBL in healthy subjects.**

| Panel 2A. Reagent Composition of 7 antibodies | | | | | | | | | |
|---|---|---|---|---|---|---|---|---|---|
| Tube Version | Label (L) position | | | | | | | | |
| | L1 | L2 | L3 | L4 | L5 | L6 | L7 | L8 | L9 |
| 1 | CD19 | CD5 | CD38 and CD81 | CD27 | - | CD180 | CD43 | | |
| 2 | CD19 | CD5 | CD38 and CD81 | CD27 | - | CD180 | CD79b | | |
| 3 | CD19 | CD5 | CD38 and CD81 | CD27 | - | CD180 | ROR1 | | |
| 4 | CD19 | CD5 | CD38 | CD27 | CD81 | CD180 | CD43 | | |
| 5 | CD19 | CD5 | CD38 | CD27 | CD81 | CD180 | CD79b | | |
| 6 | CD19 | CD5 | CD38 | CD27 | CD81 | CD180 | ROR1 | | |

| Panel 2B. Reagent Composition of 8 antibodies | | | | | | | | | |
|---|---|---|---|---|---|---|---|---|---|
| 1 | CD19 | CD5 | CD38 and CD81 | CD27 | - | CD180 | CD43 | CD79b | |
| 2 | CD19 | CD5 | CD38 and CD81 | CD27 | - | CD180 | CD79b | ROR1 | |
| 3 | CD19 | CD5 | CD38 and CD81 | CD27 | - | CD180 | ROR1 | CD43 | |
| 4 | CD19 | CD5 | CD38 | CD27 | CD81 | CD180 | CD43 | CD79b | |
| 5 | CD19 | CD5 | CD38 | CD27 | CD81 | CD180 | CD79b | ROR1 | |
| 6 | CD19 | CD5 | CD38 | CD27 | CD81 | CD180 | ROR1 | CD43 | |

| Panel 2C. Reagent Composition of 9 antibodies | | | | | | | | | |
|---|---|---|---|---|---|---|---|---|---|
| 1 | CD19 | CD5 | CD38 and CD81 | CD27 | - | CD180 | CD43 | CD79b | ROR1 |
| 2 | CD19 | CD5 | CD38 | CD27 | CD81 | CD180 | CD43 | CD79b | ROR1 |

**Table 3. Exemplary 8- to 11-label reagent compositions comprising between 9 and 11 antibody reagents for MRD detection of CLL cells and MBL cells and simultaneous assessment of their clonal nature based on restricted immunoglobulin light chain expression.**

| Panel 3A. Reagent composition of 9 antibodies | | | | | | | | | | | |
|---|---|---|---|---|---|---|---|---|---|---|---|
| Tube Version | Label (L) position | | | | | | | | | | |
| | L1 | L2 | L3 | L4 | L5 | L6 | L7 | L8 | L9 | L10 | L11 |
| 1 | CD19 | CD5 | CD38 and CD81 | CD27 | - | CD180 | CD43 | IgKappa | IgLambda | | |
| 2 | CD19 | CD5 | CD38 and CD81 | CD27 | - | CD180 | CD79b | IgKappa | IgLambda | | |
| 3 | CD19 | CD5 | CD38 and CD81 | CD27 | - | CD180 | ROR1 | IgKappa | IgLambda | | |
| 4 | CD19 | CD5 | CD38 | CD27 | CD81 | CD180 | CD43 | IgKappa | IgLambda | | |
| 5 | CD19 | CD5 | CD38 | CD27 | CD81 | CD180 | CD79b | IgKappa | IgLambda | | |
| 6 | CD19 | CD5 | CD38 | CD27 | CD81 | CD180 | ROR1 | IgKappa | IgLambda | | |

| Panel 3B. Reagent Composition of 10 antibodies | | | | | | | | | | | |
|---|---|---|---|---|---|---|---|---|---|---|---|
| 1 | CD19 | CD5 | CD38 and CD81 | CD27 | - | CD180 | CD43 | CD79b | IgKappa | IgLambda | |
| 2 | CD19 | CD5 | CD38 and CD81 | CD27 | - | CD180 | CD79b | ROR1 | IgKappa | IgLambda | |
| 3 | CD19 | CD5 | CD38 and CD81 | CD27 | - | CD180 | ROR1 | CD43 | IgKappa | IgLambda | |
| 4 | CD19 | CD5 | CD38 | CD27 | CD81 | CD180 | CD43 | CD79b | IgKappa | IgLambda | |
| 5 | CD19 | CD5 | CD38 | CD27 | CD81 | CD180 | CD79b | ROR1 | IgKappa | IgLambda | |
| 6 | CD19 | CD5 | CD38 | CD27 | CD81 | CD180 | ROR1 | CD43 | IgKappa | IgLambda | |

| Panel 3C. Reagent Composition of 11 antibodies | | | | | | | | | | | |
|---|---|---|---|---|---|---|---|---|---|---|---|
| 1 | CD19 | CD5 | CD38 and CD81 | CD27 | - | CD180 | CD43 | CD79b | ROR1 | IgKappa | IgLambda |
| 2 | CD19 | CD5 | CD38 | CD27 | CD81 | CD180 | CD43 | CD79b | ROR1 | IgKappa | IgLambda |

**Table 4. Exemplary CLL MRD / MBL reagent compositions for clear-cut discrimination between normal/reactive B-cells and both malignant CLL cells and CLL-like MBL cells and simultaneous identification of both CD45+ leukocytes and CD3+ T-cells and T-cell doublets and their discrimination of CLL MRD and CLL-like MBL cells.**

| Panel 4A. CLL MRD / MBL panel with inclusion of CD45 | | | | | | | | | | | | | |
|---|---|---|---|---|---|---|---|---|---|---|---|---|---|
| Tube Version | Label (L) position | | | | | | | | | | | | |
| | L1 | L2 | L3 | L4 | L5 | L6 | L7 | L8 | L9 | L10 | L11 | L12 | L13 |
| 1 | CD19 | CD5 | CD38 and CD81 | CD27 | - | CD180 | CD43 | CD79b | ROR1 | IgKappa | IgLambda | CD45 | |
| 2 | CD19 | CD5 | CD38 | CD27 | CD81 | CD180 | CD43 | CD79b | ROR1 | IgKappa | IgLambda | CD45 | |

| Panel 4B. CLL MRD / MBL panel with inclusion of CD45 and CD3 | | | | | | | | | | | | | |
|---|---|---|---|---|---|---|---|---|---|---|---|---|---|
| 1 | CD19 | CD5 | CD38 and CD81 | CD27 | - | CD180 | CD43 | CD79b | ROR1 | IgKappa | IgLambda | CD45 | CD3 |
| 2 | CD19 | CD5 | CD38 | CD27 | CD81 | CD180 | CD43 | CD79b | ROR1 | IgKappa | IgLambda | CD45 | CD3 |

**Table 5. Illustrating examples of the CLL MRD / MBL panels proposed for clear-cut discrimination between normal/reactive B -cells and both malignant CLL cells and CLL-like MBL cells and simultaneous identification of both CD45+ leukocytes and CD3+ T-cells and T-cell doublets and their discrimination of CLL MRD and CLL-like MBL cells with examples of fluorochrome-conjugated (panels A and B) and isotope label (panels C and D) antibody conjugates.**

| Tube Version | Fluorochrome label position | | | | | | | | | | | | |
|---|---|---|---|---|---|---|---|---|---|---|---|---|---|
| | PECy7 | BV421 | BV510 | BB515 | BV650 | PE | BV605 | PerCPCy5.5 | APC | APC705 | APC-Fire750 | BV786 | BV711 |
| Panel 5A. 11- or 12-label reagent composition with compatible fluorochrome-conjugated antibodies | | | | | | | | | | | | | |
| 1 | CD19 | CD5 | CD38 and CD81 | CD27 | - | CD180 | CD43 | CD79b | ROR1 | IgKappa | IgLambda | CD45 | |
| 2 | CD19 | CD5 | CD38 | CD27 | CD81 | CD180 | CD43 | CD79b | ROR1 | IgKappa | IgLambda | CD45 | |

| Panel 5B. 12- or 13-label reagent composition with compatible fluorochrome-conjugated antibodies | | | | | | | | | | | | | |
|---|---|---|---|---|---|---|---|---|---|---|---|---|---|
| 1 | CD19 | CD5 | CD38 and CD81 | CD27 | - | CD180 | CD43 | CD79b | ROR1 | IgKappa | IgLambda | CD45 | CD3 |
| 2 | CD19 | CD5 | CD38 | CD27 | CD81 | CD180 | CD43 | CD79b | ROR1 | IgKappa | IgLambda | CD45 | CD3 |

| Tube Version | Isotope Label position | | | | | | | | | | | | |
|---|---|---|---|---|---|---|---|---|---|---|---|---|---|
| | 89 Y | 106 Cd | 115 In | 139 La | 141 Pr | 142 Nd | 147 Sm | 151 Eu | 155 Gd | 159 Tb | 161 Dy | 165 Ho | 197 Au |
| Panel 5C. 12-label reagent composition with compatible isotope-conjugated antibodies | | | | | | | | | | | | | |
| 1 | CD19 | CD5 | CD38 | CD27 | CD81 | CD180 | CD43 | CD79b | ROR1 | Ig Kappa | Ig Lambda | CD45 | |

| Panel 5D. 13-label reagent composition with compatible isotope-conjugated antibodies | | | | | | | | | | | | | |
|---|---|---|---|---|---|---|---|---|---|---|---|---|---|
| 1 | CD19 | CD5 | CD38 | CD27 | CD81 | CD180 | CD43 | CD79b | ROR1 | Ig Kappa | Ig Lambda | CD45 | CD3 |

**Table 6. Overall pattern of expression of the CD45, CD19, CD5, CD27, CD38, CD81, CD180, ROR1, CD79b, CD43, κ and λ markers on the cell surface membrane of CLL cells vs different compartments of bone marrow and peripheral blood B-cells**

| | CD45 | CD19 | CD5 | CD27 | CD38 | CD81 | CD180 | ROR1 | CD79b | CD43 | κ | λ |
|---|---|---|---|---|---|---|---|---|---|---|---|---|
| CLL cells | +++ | +/++ | ++ | + | -/+ | -/+ | -/+ | -/++ | -/+ | + | -/+ | -/+ |
| Normal cells: | | | | | | | | | | | | |
| B-cell precursors | +/++ | +/++ | - | - | ++ | ++ | - | -/+ | - | + | - | - |
| Immature | +++ | ++ | +/++ | - | + | + | +/++ | - | + | -/+ | -/++ | -/++ |
| Naive | +++ | ++ | -/++ | - | -/+ | + | ++ | - | + | - | -/++ | -/++ |
| Unswitched memory B-cells | +++ | ++ | - | + | - | + | ++ | - | + | - | -/++ | -/++ |
| Switched memory B-cells | +++ | ++ | - | + | - | + | ++ | - | + | - | -/++ | -/++ |
| Plasma cells | ++ | -/+ | - | ++ | +++ | + | - | - | - | + | - /++^{#} | -/+++^{#} |

| | | | | | | | | | | | | |
|---|---|---|---|---|---|---|---|---|---|---|---|---|
| *CD3 is a T-cell marker systematically absent in all normal B-cell populations and CLL cells and allows to identify CD5+ non-T-cells. ^{#} κ and λ can be detected in plasma cells using intracellular staining protocols. | | | | | | | | | | | | |

The present invention is particularly suitable to be used in bone marrow samples, blood samples and other body tissues and body fluids, such as cerebrospinal fluid, saliva, tears, pleural effusions, peritoneal fluid, bronchoalveolar lavage, lymph nodes, thymus, mediastinal and abdominal masses. Blood containing samples are collected in K₂EDTA, K₃EDTA, heparin or citrate as anticoagulant. Alternatively, they can be stained fresh or after stabilization with commercially available reagents like TransfixTM.

In one embodiment of this invention, staining of samples is performed in the whole sample, after erythrocyte lysis or on mononuclear cells isolated from the whole sample using conventional techniques for staining of cell surface membrane markers plus cytoplasmic markers (such as (Ig) kappa (κ) and Ig lambda (λ) light chains), including the EuroFlow standard operating procedures for bulk lysis and for staining of cell surface membrane and cytoplasmic markers, which detailed description is available at www.EuroFlow.org.

In one embodiment of this invention, antibody reagents used to stain the above listed types of sample are conjugated with labels selected from the following list of compatible fluorochromes: BUV395, DyLight 350, BUV496, BUV563, BUV615, BUV661, BUV737, BUV805, BV421, Pacific Blue, BV480, eFluor506, BV510, BV570, BV605, BV650, BV711, BV750, BV786, BB515, FITC, CF514, PerCP, BB700, PerCP-Cy5.5, PerCP-eFluor710, BB790-P, PE, CF568, PE-Dazzle594, CF594, PE-AF610, PE-Cy5, PE-Cy5.5, PE-Cy7, APC, AF647, Spark NIR 685, APC-R700, AF700, APC-Vio770, APC-Fire810, sparkBlue550, realBlue780, realyellow586, PE-Vio615, PE-Fire640, PE-Fire700, PE-Fire810, APC-C750, R718, APC705.

In one embodiment, antibody reagents are conjugated with labels selected from the following list of compatible isotopes: 89Y, 106Cd, 110Cd, 111Cd, 112Cd, 113Cd, 114Cd, 115In, 116Cd, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 194Pt, 195Pt, 196Pt, 197Au, 198Pt, 209Bi.

As will be appreciated by a person skilled in the art, detection of CLL/MBL leukemia/lymphoma cells according to the invention can be readily performed using conventional flow cytometry, next generation flow cytometry, spectral cytometry or mass cytometry.

### EXPERIMENTAL SECTION

The invention is exemplified by the example below, which is provided for illustrating purposes and in no manner limits the scope.

### EXAMPLE 1: Analysis of a peripheral blood sample from a CLL patient treated with FCR.

Sample collection. A peripheral blood (PB) sample was collected in EDTA as anticoagulant from a CLL patient after 9 cycles of FCR (fludarabine, Cyclophosphamide, Rituximab) therapy. The PB sample was processed locally with standardized sample staining protocols, including the bulk lysis protocol for staining of 20x10⁶ cells, as well as with a standardized cytometer set-up, calibration and staining procedures developed by the EuroFlow Consortium which have been described previously and that are available at https://euroflow.org/protocols (Kalina et al. 2012; Van Dongen et al. 2012).

Briefly, 10 ml of the PB sample plus ammonium chloride lysing solution were mixed at a 1/5 ratio in a 50ml Falcon tube and incubated for 15 min in a sample-shaker device. Afterward, the sample was centrifuged at 800g for 10 min and the supernatant discarded using a Pasteur pipette without disturbing the cell pellet. Upon discarding the supernatant, the tube was refilled with PBS containing 0.09% NaN₃ and 0.5 % BSA to a final volume of 50 ml and centrifuged once again at 800g (5 min). Without disturbing the cell pellet, the supernatant was discarded, and the cell pellet was resuspended in 2 mL of PBS with 0.09% NaN₃ and 0.5 % BSA. Then, the cells were transferred to a 5 mL polystyrene round-bottom Falcon tube ("FACS tube") in a volume of 300 µl/tube. Such volume was completed with PBS containing 0.09% NaN3 and 0.5 % BSA to reach 2ml (final volume), gently mixed and centrifuged at 540g for 5min; then, the supernatant was removed using a Pasteur pipette without disturbing the cell pellet. This procedure was repeated twice. The final cell concentration was adjusted with PBS containing 0.09% NaN3 and 0.5 % BSA to 2 x 10⁶ cells/µL and around 100 µL (i.e. 20 million cells) of the final cell suspension/sample were used to be stained in one tube and measured in the flow cytometer.

**Staining of the sample.** Two 50pl-aliquots of the above processed specimen samples were subsequently stained with the appropriate volume (saturating concentration) of monoclonal antibody combination (reagent composition) of the CLL-MRD panel being the subject of the current invention (Table 1A; Figure 1).

In the first step, an admixture of monoclonal antibodies (Mabs) against selected surface markers was added: CD19 (J3.119) - phycoerythrin-cyanine 7 (PECy7) (Beckmann Coulter); CD5 (L17F12) - Brilliant Violet 421 (BV421) (Biolegend); CD38 (HIT2) - Brilliant Violet 510 (BV510) (Biolegend); CD81 (JS81) - BV510 (Biolegend); CD27 (M-T271) - Brilliant Blue 515 (BB515) (Becton Dickinson Biosciences, BD); and CD180 - phycoerythrin (PE) (Biolegend); (i.e. the 6-label, 6-antibody reagent composition 1 in Table 1A).

After the cells and antibody reagents directed against cell surface markers were mixed well, they were incubated for 30 min at RT protected from light. After this incubation, 2 mL of PBS containing 0.09% NaN₃ and 0.5 % BSA was added to the cell pellet, mixed well and centrifuged for 5 min at 540g. Then, the supernatant was discarded using a Pasteur pipette or vacuum system without disturbing the cell pellet, and approximately 50µL of residual volume was left in each tube. Upon mixing well the residual volume, the cell pellet was resuspended in 200µL PBS with 0.09% NaN₃ and 0.5% BSA, and immediately measured in a BD FACS Lyric or BD LSRFortessa X-20 flow cytometers equipped with 3 lasers and 12 fluorescence detectors. Approximately 35 million cells were acquired.

### Data analysis and Results

First, total PB events are displayed on a FCS vs SSC dot plot to select those cells corresponding to cellular events present in PB (Figure 1A). Next, on a CD19 vs. SSC dot plot total B cells are gated to only include the events with CD19 expression and low side scatter (SSC) values (Figure 1B). Then, the selected events are displayed on a FSC-A vs FSC-H dot plot where only those events positioned in the diagonal are selected to exclude cell doublets and triplets (i.e., multiplets) (Figure 1C). Subsequently, the selected total (single) B cell events are shown in a CD5 vs. CD27 dot plot, where CD5 and CD27 double positive events potentially including CD5+ CD27+ CLL cells (together with CD5 and CD27 double positive normal B cell events) are selected (Figure 1D). Afterwards, CD5+CD27+ B cells are plotted on an APS1 (principal component analysis graphical representation of principal component 1 vs principal component 2) diagram plot or canonical analysis (CA) plot based on the expression of CD180, CD38 and CD81 on the individual CD5+ CD27+ B cells (Figure 1E), and the different clusters of events gated to identify the cluster of CLL cells showing lower CD38, CD81 and CD180 expression levels as illustrated for CD180 in the CD180 vs CD27 dot plot shown in Figure 1F.

### EXAMPLE 2: Analysis of bone marrow and peripheral blood samples

Sample collection. For MFC analyses, a total of 126 follow-up bone marrow (BM, n= 25) and peripheral blood (PB, n=78) samples were collected in EDTA as anticoagulant at different time points following 3, 6 and 9 cycles of different therapies of with possible multiple samples collected from the same patient. All BM and PB samples were processed locally with standardized sample staining protocols, including bulk lysis protocol for staining ≥ 5x10⁶ cells in follow-up CLL/MBL samples, as well as with standardized cytometer set-up, calibration and staining procedures developed by the EuroFlow Consortium which have been described previously and that are available at https://euroflow.org/protocols (Kalina et al. 2012; Van Dongen et al. 2012).

Briefly, 2 ml of each sample plus 50 mL of ammonium chloride lysing solution were mixed in a 50ml Falcon tube and incubated for 15 min in a sample-shaker device. Afterward, the sample was centrifuged at 800g for 10 min and the supernatant discarded using a Pasteur pipette without disturbing the cell pellet. Upon discarding the supernatant, the tube was refilled with PBS containing 0.09% NaN₃ and 0.5 % BSA to a final volume of 50 ml and centrifuged once again at 800g (5 min). Without disturbing the cell pellet, the supernatant was discarded, and the cell pellet was resuspended in 2 mL of PBS with 0.09% NaN₃ and 0.5 % BSA. Then, the cells were transferred to a 5 mL polystyrene round-bottom Falcon tube ("FACS tube") in a volume of 300 µl/tube. Such volume was completed with PBS containing 0.09% NaN3 and 0.5 % BSA to reach 2ml (final volume), gently mixed and centrifuged at 540g for 5min; then, the supernatant was removed using a Pasteur pipette without disturbing the cell pellet. This procedure was repeated twice. The final cell concentration was adjusted with PBS containing 0.09% NaN₃ and 0.5 % BSA to 5 x 10⁵ cells/µL and around 100 µL (i.e. 10 million cells) of the final cell suspension/sample were used to be stained in one tube and measured in the flow cytometer.

**Staining of the sample.** Two 100pl-aliquots of the above processed specimen samples were subsequently stained with the appropriate volume (saturating concentration) of monoclonal antibody combination (reagent composition) of the CLL-MRD panel being the subject of the current invention (Table 5B).

In the first step, an admixture of monoclonal antibodies (Mabs) against selected surface markers was added: CD19 (J3.119) - phycoerythrin-cyanine 7 (PECy7) (Beckmann Coulter); CD5 (L17F12) - Brilliant Violet 421 (BV421) (Biolegend); CD38 (HIT2) - Brilliant Violet 510 (BV510) (Biolegend); CD81 (JS81) - BV510 (Biolegend); CD27 (M-T271) - Brilliant Blue 515 (BB515) (Becton Dickinson Biosciences, BD); CD180 - phycoerythrin (PE) (Biolegend); CD43 (1G10) - Brilliant Violet 605 (BV605) (BD); CD79b (SN8) - peridinin-chlorophyll protein-Cyanine5.5 (PerCP-Cy5.5) (BD); ROR1 (2A2) - allophycocyanine (APC) (Biolegend); IgKappa (IgK) - allophycocyanine 750 (APC750) (Cytognos); IgLambda (IgL) - allophycocyanine-Fire 750 (APC-Fire750) (Biolegend); CD45 (HI30) - Brilliant Violet 786 (BV786) (Biolegend); CD3 (SK7) - Brilliant Violet 711 (BV711) (Biolegend) (i.e. the 12-label, 13-antibody reagent composition 1 in Table 5B).

After the cells and antibody reagents directed against cell surface markers were mixed well, they were incubated for 30 min at RT protected from light. After this incubation, 2 mL of PBS containing 0.09% NaN₃ and 0.5 % BSA was added to the cell pellet, mixed well and centrifuged for 5 min at 540g. Then, the supernatant was discarded using a Pasteur pipette or vacuum system without disturbing the cell pellet, and approximately 50 µL of residual volume was left in each tube. Upon mixing well the residual volume, the cell pellet was resuspended in 200µL PBS with 0.09% NaN₃ and 0.5% BSA, and immediately measured in a BD FACS Lyric or BD LSRFortessa X-20 flow cytometers equipped with 3 lasers and 12 fluorescence detectors. Approximately 1.3 million cells were acquired.

### Data analysis and Results

First, total BM or PB events displayed on a FSC vs SSC dot plot (BM samples) or a CD45 vs SSC (PB samples) to select those cells corresponding to cellular events present in BM and CD45+ leucocytes present in PB, respectively (Figure 2A and 2B, respectively). Next, on a CD19 vs. SSC dot plot total B cells are gated to only include the events with CD19 expression and low side scatter (SSC) values (Figure 2C). Then, the selected events are displayed on a CD3 vs. SSC dot plot, where only those CD3-negative events are gated (Figure 2D). Next, the selected events are shown on an FSC-A vs FSC-H dot plot where only those events positioned in the diagonal are selected to exclude cell doublets and triplets (i.e., multiplets) (Figure 2E). In the following step, the selected total B cell events are shown in a CD5 vs. CD27 dot plot, where CD5 and CD27 double positive B cell events potentially including CD5+ CD27+ CLL cells (together with CD5 and CD27 double positive normal B cell events) are selected (Figure 2F). Afterwards, total CD5+CD27+ B cells are plotted on an APS1 (principal component analysis graphical representation of principal component 1 vs principal component 2) diagram plot or a canonical analysis (CA) plot, based on the expression of CD180, CD38 and CD81 on the individual CD5+ CD27+ B cells (Figure 2G), and the different clusters of events gated to identify the cluster of CLL cells showing lower CD38, CD81 and CD180 expression levels as illustrated in a CD180 vs CD43 dot plot (Figure 2H) and a CD38+CD81 vs ROR1 dot plot (Figure 2I). Finally, the CLL nature of the gated CLL events based on their typical phenotype for ROR1 (positive)(Figure 2I), CD79b (negative to low expression), CD43 (positive) (Figure 2H), and negative or dim expression of surface immunoglobulin light chain (restricted to) kappa or lambda (Figure 2J).

### REFERENCES

1. Hallek M, Cheson BD, Catovsky D, Caligaris-Cappio F, Dighiero G, Döhner H, et al. iwCLL guidelines for diagnosis, indications for treatment, response assessment, and supportive management of CLL. Blood. 2018 Jun 21;131(25):2745-60.
2. Bottcher S, Ritgen M, Fischer K, Stilgenbauer S, Busch RM, Fingerle-Rowson G, et al. Minimal residual disease quantification is an independent predictor of progression-free and overall survival in chronic lymphocytic leukemia: a multivariate analysis from the randomized GCLLSG CLL8 trial. J Clin Oncol. 2012;30(9):980-8.
3. Thompson PA, Srivastava J, Peterson C, Strati P, Jorgensen JL, Hether T, et al. Minimal residual disease undetectable by next-generation sequencing predicts improved outcome in CLL after chemoimmunotherapy. Blood. 2019 Nov 28;134(22):1951-9.
4. Hengeveld PJ, van der Klift MY, Kolijn PM, Davi F, Kavelaars FG, de Jonge E, et al. Detecting measurable residual disease beyond 10-4 by an IGHV leader-based NGS approach improves prognostic stratification in CLL. Blood. 2023 Feb 2;141(5):519-28.
5. Letestu R, Dahmani A, Boubaya M, Baseggio L, Campos L, Chatelain B, et al. Prognostic value of high-sensitivity measurable residual disease assessment after front-line chemoimmunotherapy in chronic lymphocytic leukemia. Leukemia. 2021 Jun;35(6):1597-609.
6. Flores-Montero J, Sanoja-Flores L, Paiva B, Puig N, García-Sánchez O, Böttcher S, et al. Next Generation Flow for highly sensitive and standardized detection of minimal residual disease in multiple myeloma. Leukemia. 2017 Oct 10;31(10):2094-103.
7. Theunissen P, Mejstrikova E, Sedek L, van der Sluijs-Gelling AJ, Gaipa G, Bartels M, et al. Standardized flow cytometry for highly sensitive MRD measurements in B-cell acute lymphoblastic leukemia. Blood. 2017 Jan 19;129(3):347-57.
8. Freedman AS, Boyd AW, Bieber FR, Daley J, Rosen K, Horowitz JC, et al. Normal cellular counterparts of B cell chronic lymphocytic leukemia. Blood. 1987 Aug;70(2):418-27.
9. Drexler HG, Brenner MK, Wimperis JZ, Gignac SM, Janossy G, Prentice HG, et al. CD5-positive B cells after T cell depleted bone marrow transplantation. Clin Exp Immunol. 1987 Jun;68(3):662-8.
10. Almasri NM, Duque RE, Iturraspe J, Everett E, Braylan RC. Reduced expression of CD20 antigen as a characteristic marker for chronic lymphocytic leukemia. Am J Hematol. 1992;40(4):259-63.
11. Mason DY, Stein H, Gerdes J, Pulford KA, Ralfkiaer E, Falini B, et al. Value of monoclonal anti-CD22 (p135) antibodies for the detection of normal and neoplastic B lymphoid cells. Blood. 1987 Mar;69(3):836-40.
12. Ludescher C, Gattringer C, Weger AR, Drach J, Thaler J, Bitschmann R, et al. Surface immunoglobulin density in the differential diagnosis of B-cell chronic lymphocytic leukemia and leukemic immunocytoma. Leuk Res. 1992;16(2):191-6.
13. Garcia Vela J, Delgado I, Benito L, Monteserin M, Garcia Alonso L, Somolinos N, et al. CD79b expression in B cell chronic lymphocytic leukemia: its implication for minimal residual disease detection. Leukemia. 1999 Oct;13(10):1501-5.
14. Roliński J, Rupniewska ZM, Dmoszyska A, Wasik-Szczepanek E, Bojarska-Junak A. [CD43 in B-cell chronic lymphocytic leukemia]. Pol Arch Med Wewn. 1999 Sep;102(3):753-62.
15. Wormsley SB, Rai KR, Gale RP. Analysis of treatment response in chronic lymphocytic leukemia: new approaches. Nouv Rev Fr Hematol. 1988;30(5-6):413-7.
16. Barrena S, Almeida J, Yunta M, Lopez A, Fernandez-Mosteirin N, Giralt M, et al. Aberrant expression of tetraspanin molecules in B-cell chronic lymphoproliferative disorders and its correlation with normal B-cell maturation. Leukemia. 2005;19(8):1376-83.
17. Rawstron AC, Villamor N, Ritgen M, Bottcher S, Ghia P, Zehnder JL, et al. International standardized approach for flow cytometric residual disease monitoring in chronic lymphocytic leukaemia. Leukemia. 2007;21(5):956-64.
18. Raponi S, Della Starza I, De Propris MS, Del Giudice I, Mauro FR, Marinelli M, et al. Minimal residual disease monitoring in chronic lymphocytic leukaemia patients. A comparative analysis of flow cytometry and ASO IgH RQ-PCR. Br J Haematol. 2014 Aug;166(3):360-8.
19. Rawstron AC, Bö Ttcher S, Letestu R, Villamor N, Fazi C, Kartsios H, et al. Improving efficiency and sensitivity: European Research Initiative in CLL (ERIC) update on the international harmonised approach for flow cytometric residual disease monitoring in CLL. Leukemia. 2013;27:142-9.
20. Farren TW, Giustiniani J, Fanous M, Liu F, Macey MG, Wright F, et al. Minimal residual disease detection with tumor-specific CD160 correlates with event-free survival in chronic lymphocytic leukemia. Blood Cancer J. 2015 Jan 23;5(1):e273.
21. De Propris MS, Intoppa S, Milani ML, Mariglia P, Nardacci MG, Peragine N, et al. ROR1 is an accurate and reliable marker of minimal residual disease in chronic lymphocytic leukaemia. Br J Haematol. 2020 Sep;190(6):e346-9.
22. Dongen JJMV, Vale JAO de MCE, MONTERO JAF, Parra JMA, Velden VHJVD, Böttcher S, et al. Methods, reagents and kits for detecting minimal residual disease WO2013187765A2,
23. Rawstron AC, Fazi C, Agathangelidis A, Villamor N, Letestu R, Nomdedeu J, et al. A complementary role of multiparameter flow cytometry and high-throughput sequencing for minimal residual disease detection in chronic lymphocytic leukemia: an European Research Initiative on CLL study. Leukemia. 2016;30(4):929-36.
24. Sartor MM, Gottlieb DJ. A single tube 10-color flow cytometry assay optimizes detection of minimal residual disease in chronic lymphocytic leukemia. Cytometry B Clin Cytom. 2013 Mar;84(2):96-103.
25. Marti GE, Rawstron AC, Ghia P, Hillmen P, Houlston RS, Kay N, et al. Diagnostic criteria for monoclonal B-cell lymphocytosis. Br J Haematol. 2005;130(3):325-32.
26. Sarasua SM, Vogt RF, Middleton DC, Slade BA, McGeehin MA, Lybarger JA. CLL-like B-cell phenotypes detected in superfund studies: Epidemiologic methods and findings. In: Proceedings of a USPHS Workshop on Laboratory Approaches to Determining the Role of Environmental Exposures as Risk Factors for B-cell Chronic Lymphocytic Leukemia and Other B-cell Lymphoproliferative Disorders. Atlanta, GA.: U.S. Department of Health and Human Services, Public Health Service, Centers for Disease Control and Prevention : Food and Drug Administration : Agency for Toxic Substances and Disease Registry : National Institutes of Health; 1997. p. 7-18.
27. Rawstron AC, Green MJ, Kuzmicki A, Kennedy B, Fenton JAL, Evans PAS, et al. Monoclonal B lymphocytes with the characteristics of "indolent" chronic lymphocytic leukemia are present in 3.5% of adults with normal blood counts. Blood. 2002;
28. Ghia P, Prato G, Scielzo C, Stella S, Geuna M, Guida G, et al. Monoclonal CD5+ and CD5- B-lymphocyte expansions are frequent in the peripheral blood of the elderly. Blood. 2004;
29. Rachel JM, Zucker ML, Fox CM, Plapp FV, Menitove JE, Abbasi F, et al. Monoclonal B-cell lymphocytosis in blood donors. Br J Haematol. 2007 Dec;139(5):832-6.
30. Nieto WG, Almeida J, Romero A, Teodosio C, López A, Henriques AF, et al. Increased frequency (12%) of circulating chronic lymphocytic leukemia-like B-cell clones in healthy subjects using a highly sensitive multicolor flow cytometry approach. Blood. 2009;114(1):33-7.
31. Rawstron AC, Ssemaganda A, de Tute R, Doughty C, Newton D, Vardi A, et al. Monoclonal B-cell lymphocytosis in a hospital-based UK population and a rural Ugandan population: a cross-sectional study. Lancet Haematol. 2017;
32. Oliva-Ariza G, Fuentes-Herrero B, Carbonell C, Lecrevisse Q, Pérez-Pons A, Torres-Valle A, et al. High frequency of low-count monoclonal B-cell lymphocytosis in hospitalized COVID-19 patients. Blood. 2023 Jan 19; 141(3):309-14.

## Claims

1. A reagent composition for the cytometric detection of minimal residual disease (MRD) in chronic lymphocytic leukemia (CLL), the reagent composition comprising a panel of at least six antibodies conjugated to a detectable label, wherein the panel comprises antibodies directed against the markers CD180, CD38, CD81, CD19, CD27 and CD5, and wherein at least the antibodies against the markers CD5, CD19, CD27 and CD180 are each conjugated to a distinct detectable label.

2. Reagent composition according to claim 1, wherein the antibodies against the markers CD38 and CD81 are conjugated to the same detectable label.

3. Reagent composition according to claim 1 or 2, wherein the panel further comprises antibodie(s) conjugated to a detectable label wherein the antibodies are directed against one or more markers selected from CD43, CD79b and ROR1.

4. Reagent composition according to claim 3, comprising conjugated antibodie(s) directed against the markers CD43, CD79b and ROR1.

5. Reagent composition according to any one of the preceding claims, wherein the panel further comprises a set of antibodies directed against human immunoglobulin (Ig) kappa and Ig lambda light chains, wherein each antibody within the set is conjugated to a distinct detectable label.

6. Reagent composition according to any one of the preceding claims, wherein the panel further comprises an antibody directed against CD45 and wherein the antibody is conjugated to a detectable label.

7. Reagent composition according to any one of the preceding claims, wherein the panel further comprises an antibody directed against CD3 and wherein the antibody is conjugated to a detectable label.

8. Reagent composition according to any one of the preceding claims, wherein the composition does not contain a conjugated antibody directed against CD20 and/or a conjugated antibody directed against CD200.

9. Reagent composition according to any one of the preceding claims, comprising or consisting of conjugated antibodies against one of the following sets of markers:
(i) CD19, CD5, CD38, CD81, CD27, CD180 and CD43;
(ii) CD19, CD5, CD38, CD81, CD27, CD180 and CD79b;
(iii) CD19, CD5, CD38, CD81, CD27, CD180 and ROR1;
(iv) CD19, CD5, CD38, CD81, CD27, CD180, CD43 and CD79b;
(v) CD19, CD5, CD38, CD81, CD27, CD180, CD79b and ROR1;
(vi) CD19, CD5, CD38, CD81, CD27, CD180, ROR1 and CD43;
(vii) CD19, CD5, CD38, CD81, CD27, CD180, CD43, CD79b and ROR1;
(viii) CD19, CD5, CD38, CD81, CD27, CD180, CD43, IgKappa and IgLambda;
(ix) CD19, CD5, CD38, CD81, CD27, CD180, CD79b, IgKappa and IgLambda;
(x) CD19, CD5, CD38, CD81, CD27, CD180, ROR1, IgKappa and IgLambda.
(xi) CD19, CD5, CD38, CD81, CD27, CD180, CD43, CD79b, IgKappa and IgLambda;
(xii) CD19, CD5, CD38, CD81, CD27, CD180, CD79b, ROR1, IgKappa and IgLambda;
(xiii) CD19, CD5, CD38, CD81, CD27, CD180, ROR1, CD43, IgKappa and IgLambda;
(xiv) CD19, CD5, CD38, CD81, CD27, CD180, CD43, CD79b, IgKappa and IgLambda;
(xv) CD19, CD5, CD38, CD81, CD27, CD180, CD43, CD79b, ROR1, IgKappa, IgLambda and CD45;
(xvi) CD19, CD5, CD38, CD81, CD27, CD180, CD43, CD79b, ROR1, IgKappa, IgLambda, CD45 and CD3.

10. Reagent composition according to any one of the preceding claims, wherein the antibodies are conjugated to a fluorochrome.

11. Reagent composition according to any one claims 1-9, wherein the antibodies are conjugated to a metal isotope, preferably selected from the group consisting of 89Y, 106Cd, 110Cd, 111Cd, 112Cd, 113Cd, 114Cd, 115In, 116Cd, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 194Pt, 195Pt, 196Pt, 197Au, 198Pt and 209Bi.

12. The *in vitro* use of a reagent composition according to any one of the preceding claims as CLL-MRD detection reagent.

13. A diagnostic kit for flow cytometric detection of minimal residual disease (MRD) in chronic lymphocytic leukemia (CLL), comprising at least one reagent composition according to claims 1-11, optionally together with instructions for use, buffer, and/or control samples.

14. A cytometric method for detecting minimal residual disease (MRD) in chronic lymphocytic leukemia (CLL), comprising the steps of:
(i) contacting an aliquot of a biological sample comprising leukocytes with a reagent composition according to any one of claims 1-11,
(ii) analyzing leukocytes in said aliquot in a flow or mass cytometer;
(iii) gating on cells for expression of the selected markers detected by the conjugated antibodies present in the reagent composition; and
(iv) distinguishing between normal and malignant cells, based on the expression profile of the multiple markers, preferably wherein step (iv) comprises discriminating between normal/reactive CD5+ CD27-/+ CD180hi B-lymphocytes and CD5+ CD27+ CD 180-/low CLL or CLL-like MBL cells..

15. Method according to claim 14, wherein the sample is selected from the group consisting of (fresh or stabilized) blood, bone marrow (BM), cerebrospinal fluid, saliva, tears, pleural effusions, peritoneal fluid, bronchoalveolar lavage, thymus, lymph nodes, mediastinal and abdominal masses, and spleen specimen, preferably wherein the sample is a blood or BM sample.

16. Method according to claim 14 or 15, wherein step (iv) involves multivariate analysis, preferably principal component analysis (PCA) and/or canonical analysis.

## Patentansprüche

1. Reagenzzusammensetzung für einen zytometrischen Nachweis von minimaler Resterkrankung (MRD) bei chronischer lymphatischer Leukämie (CLL), wobei die Reagenzzusammensetzung ein Panel von mindestens sechs Antikörpern umfasst, konjugiert mit einer nachweisbaren Markierung, wobei das Panel Antikörper umfasst, die gegen die Marker CD180, CD38, CD81, CD19, CD27 und CD5 gerichtet sind, und wobei mindestens die Antikörper gegen die Marker CD5, CD19, CD27 und CD180 jeweils mit einer verschieden nachweisbaren Markierung konjugiert sind.

2. Reagenzzusammensetzung nach Anspruch 1, wobei die Antikörper gegen die Marker CD38 und CD81 mit derselben nachweisbaren Markierung konjugiert sind.

3. Reagenzzusammensetzung nach Anspruch 1 oder 2, wobei das Panel ferner Antikörper umfasst, konjugiert mit einer nachweisbaren Markierung, wobei der/die Antikörper gegen einen oder mehrere Marker gerichtet ist/sind, ausgewählt aus CD43, CD79b und ROR1.

4. Reagenzzusammensetzung nach Anspruch 3, umfassend konjugierte/n Antikörper, gerichtet gegen die Marker CD43, CD79b und ROR1.

5. Reagenzzusammensetzung nach einem der vorhergehenden Ansprüche, wobei das Panel ferner einen Satz von Antikörpern umfasst, gerichtet gegen leichte Ketten von humanem Immunglobulin (Ig)-Kappa und Ig-Lambda, wobei jeder Antikörper innerhalb des Satzes mit einer verschieden nachweisbaren Markierung konjugiert ist.

6. Reagenzzusammensetzung nach einem der vorhergehenden Ansprüche, wobei das Panel ferner einen Antikörper umfasst, gerichtet gegen CD45, und wobei der Antikörper mit einer nachweisbaren Markierung konjugiert ist.

7. Reagenzzusammensetzung nach einem der vorhergehenden Ansprüche, wobei das Panel ferner einen Antikörper umfasst, gerichtet gegen CD3, und wobei der Antikörper mit einer nachweisbaren Markierung konjugiert ist.

8. Reagenzzusammensetzung nach einem der vorhergehenden Ansprüche, wobei die Zusammensetzung keinen konjugierten Antikörper, gerichtet gegen CD20, und/oder keinen konjugierten Antikörper, gerichtet gegen CD200, enthält.

9. Reagenzzusammensetzung nach einem der vorhergehenden Ansprüche, umfassend oder bestehend aus konjugierten Antikörpern gegen einen der folgenden Sätze von Markern:
(i) CD19, CD5, CD38, CD81, CD27, CD180 und CD43;
(ii) CD19, CD5, CD38, CD81, CD27, CD180 und CD79b;
(iii) CD19, CD5, CD38, CD81, CD27, CD180 und ROR1;
(iv) CD19, CD5, CD38, CD81, CD27, CD180, CD43 und CD79b;
(v) CD19, CD5, CD38, CD81, CD27, CD180, CD79b und RORl;
(vi) CD19, CD5, CD38, CD81, CD27, CD180, ROR1 und CD43;
(vii) CD19, CD5, CD38, CD81, CD27, CD180, CD43, CD79b und RORl;
(viii) CD19, CD5, CD38, CD81, CD27, CD180, CD43, lgKappa und lgLambda;
(ix) CD19, CD5, CD38, CD81, CD27, CD180, CD79b, lgKappa und lgLambda;
(x) CD19, CD5, CD38, CD81, CD27, CD180, RORl, lgKappa und lgLambda.
(xi) CD19, CD5, CD38, CD81, CD27, CD180, CD43, CD79b, lgKappa und lgLambda;
(xii) CD19, CD5, CD38, CD81, CD27, CD180, CD79b, RORl, lgKappa und IgLambda;
(xiii) CD19, CD5, CD38, CD81, CD27, CD180, RORl, CD43, IgKappa und IgLambda;
(xiv) CD19, CD5, CD38, CD81, CD27, CD180, CD43, CD79b, lgKappa und lgLambda;
(xv) CD19, CD5, CD38, CD81, CD27, CD180, CD43, CD79b, RORl, lgKappa, lgLambda und CD45;
(xvi) CD19, CD5, CD38, CD81, CD27, CD180, CD43, CD79b, RORl, lgKappa, lgLambda, CD45 und CD3.

10. Reagenzzusammensetzung nach einem der vorhergehenden Ansprüche, wobei die Antikörper mit einem Fluorochrom konjugiert sind.

11. Reagenzzusammensetzung nach einem der Ansprüche 1-9, wobei die Antikörper mit einem Metallisotop konjugiert sind, vorzugsweise ausgewählt aus der Gruppe bestehend aus 89Y, 106Cd, 110Cd, 111Cd, 112Cd, 113Cd, 114Cd, 115In, 116Cd, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 194Pt, 195Pt, 196Pt, 197Au, 198Pt und 209Bi.

12. *In-vitro-*Verwendung einer Reagenzzusammensetzung nach einem der vorhergehenden Ansprüche als CLL-MRD-Nachweisreagenz.

13. Diagnosekit für einen durchflusszytometrischen Nachweis von minimaler Resterkrankung (MRD) bei chronischer lymphatischer Leukämie (CLL), umfassend mindestens eine Reagenzzusammensetzung nach den Ansprüchen 1-11, optional zusammen mit einer Gebrauchsanweisung, zu Anwendung, Puffer und/oder Kontrollproben.

14. Zytometrisches Verfahren zum Nachweis von minimaler Resterkrankung (MRD) bei chronischer lymphatischer Leukämie (CLL), umfassend die Schritte von:
(i) Inkontaktbringen eines Aliquots einer biologischen Probe, umfassend Leukoyten, mit einer Reagenzzusammensetzung nach einem der Ansprüche 1-11,
(ii) Analysieren von Leukozyten in dem Aliquot in einem Durchfluss- oder Massenzytometer;
(iii) Gaten von Zellen zur Expression der ausgewählten Marker, nachgewiesen durch die konjugierten Antikörper, die in der Reagenzzusammensetzung vorhanden sind; und
(iv) Unterscheiden zwischen normalen und malignen Zellen auf der Basis des Expressionsprofils der mehreren Marker, vorzugsweise wobei Schritt (iv) ein Abgrenzen zwischen normalen/reaktiven CD5+ CD27-/+ CD180hi-B-Lymphozyten und CD5+ CD27+ CD180-/niedrig CLL oder CLL-artigen MBL-Zellen umfasst.

15. Verfahren nach Anspruch 14, wobei die Probe ausgewählt ist aus der Gruppe bestehend aus (frischen oder stabilisierten) Blut-, Knochenmark-(BM-), Hirn-Rückenmarksflüssigkeits-, Speichel-,Tränen-, Pleuraeffusions-, Peritonealflüssigkeits-, bronchoalveoläre Lavage-, Thymus-, Lymphknoten-, mediastinale und abdominale Massen- und Milzpräparaten, vorzugsweise wobei die Probe eine Blut- oder BM-Probe ist.

16. Verfahren nach Anspruch 14 oder 15, wobei Schritt (iv) eine multivariate Analyse, vorzugsweise eine Hauptkomponentenanalyse (PCA) und/oder eine kanonische Analyse beinhaltet.

## Revendications

1. Composition de réactifs pour la détection cytométrique de la maladie résiduelle minimale (MRM) dans la leucémie lymphoïde chronique (LLC), la composition de réactifs comprenant un panel d'au moins six anticorps conjugués à un marqueur détectable, dans lequel le panel comprend des anticorps dirigés contre les marqueurs CD180, CD38, CD81, CD19, CD27 et CD5, et dans lequel au moins les anticorps dirigés contre les marqueurs CD5, CD19, CD27 et CD180 sont conjugués chacun à un marqueur détectable distinct.

2. Composition de réactifs selon la revendication 1, dans laquelle les anticorps dirigés contre les marqueurs CD38 et CD81 sont conjugués au même marqueur détectable.

3. Composition de réactifs selon la revendication 1 ou la revendication 2, dans laquelle le panel comprend en outre un ou des anticorps conjugués à un marqueur détectable, les anticorps étant dirigés contre un ou plusieurs marqueurs choisis parmi CD43, CD79b et ROR1.

4. Composition de réactifs selon la revendication 3, comprenant des anticorps conjugués dirigés contre les marqueurs CD43, CD79b et ROR1.

5. Composition de réactifs selon l'une quelconque des revendications précédentes, dans laquelle le panel comprend en outre un ensemble d'anticorps dirigés contre les chaînes légères kappa et lambda des immunoglobulines (Ig) humaines, dans lequel chaque anticorps de l'ensemble est conjugué à un marqueur détectable distinct.

6. Composition de réactifs selon l'une quelconque des revendications précédentes, dans laquelle le panel comprend en outre un anticorps dirigé contre CD45, et dans laquelle l'anticorps est conjugué à un marqueur détectable.

7. Composition de réactifs selon l'une quelconque des revendications précédentes, dans laquelle le panel comprend en outre un anticorps dirigé contre CD3, et dans laquelle l'anticorps est conjugué à un marqueur détectable.

8. Composition de réactifs selon l'une quelconque des revendications précédentes, dans laquelle la composition ne contient pas d'anticorps conjugué dirigé contre CD20 et/ou d'anticorps conjugué dirigé contre CD200.

9. Composition de réactifs selon l'une quelconque des revendications précédentes, comprenant ou consistant en des anticorps conjugués contre l'un des ensembles de marqueurs suivants :
(i) CD19, CD5, CD38, CD81, CD27, CD180 et CD43 ;
(ii) CD19, CD5, CD38, CD81, CD27, CD180 et CD79b ;
(iii) CD19, CD5, CD38, CD81, CD27, CD180 et ROR1 ;
(iv) CD19, CD5, CD38, CD81, CD27, CD180, CD43 et CD79b ;
(v) CD19, CD5, CD38, CD81, CD27, CD180, CD79b et ROR1 ;
(vi) CD19, CD5, CD38, CD81, CD27, CD180, ROR1 et CD43 ;
(vii) CD19, CD5, CD38, CD81, CD27, CD180, CD43, CD79b et ROR1 ;
(viii) CD19, CD5, CD38, CD81, CD27, CD180, CD43, IgKappa et IgLambda ;
(ix) CD19, CD5, CD38, CD81, CD27, CD180, CD79b, IgKappa et IgLambda ;
(x) CD19, CD5, CD38, CD81, CD27, CD180, ROR1, IgKappa et IgLambda ;
(xi) CD19, CD5, CD38, CD81, CD27, CD180, CD43, CD79b, IgKappa et IgLambda ;
(xii) CD19, CD5, CD38, CD81, CD27, CD180, CD79b, ROR1, IgKappa et IgLambda ;
(xiii) CD19, CD5, CD38, CD81, CD27, CD180, ROR1, CD43, IgKappa et IgLambda ;
(xiv) CD19, CDS, CD38, CD81, CD27, CD180, CD43, CD79b, IgKappa et IgLambda ;
(xv) (xv) CD19, CD5, CD38, CD81, CD27, CD180, CD43, CD79b, ROR1, IgKappa, IgLambda et CD45 ;
(xvi) CD19, CDS, CD38, CD81, CD27, CD180, CD43, CD79b, ROR1, IgKappa, IgLambda, CD45 et CD3.

10. Composition de réactifs selon l'une quelconque des revendications précédentes, dans laquelle les anticorps sont conjugués à un fluorochrome.

11. Composition de réactifs selon l'une quelconque des revendications 1 à 9, dans laquelle les anticorps sont conjugués à un isotope métallique, de préférence choisi dans le groupe constitué par les ⁸⁹Y, ¹⁰⁶Cd, ¹¹⁰Cd, ¹¹¹Cd, ¹¹²Cd, ¹¹³Cd, ¹¹⁴Cd, ¹¹⁵In, ¹¹⁶Cd, ¹³⁹La, ¹⁴¹Pr, ¹⁴²Nd, ¹⁴³Nd, ¹⁴⁴Nd, ¹⁴⁵Nd, ¹⁴⁶Nd, ¹⁴⁷Sm, ¹⁴⁸Nd, ¹⁴⁹Sm, ¹⁵⁰Nd, ¹⁵¹Eu, ¹⁵²Sm, ¹⁵³Eu, ¹⁵⁴Sm, ¹⁵⁵Gd, ¹⁵⁶Gd, ¹⁵⁸Gd, ¹⁵⁹Th, ¹⁶⁰Gd, ¹⁶¹Dy, ¹⁶²Dy, ¹⁶³Dy, ¹⁶⁴Dy, ¹⁶⁵Ho, ¹⁶⁶Er, ¹⁶⁷Er, ¹⁶⁸Er, ¹⁶⁹Tm, ¹⁷⁰Er, ¹⁷¹Yb, ¹⁷²Yb, ¹⁷³Yb, ¹⁷⁴Yb, ¹⁷⁵Lu, ¹⁷⁶Yb, ¹⁹⁴Pt, ¹⁹⁵Pt, ¹⁹⁶Pt, ¹⁹⁷Au, 198pt et ²⁰⁹Bi.

12. Utilisation *in vitro* d'une composition de réactifs selon l'une quelconque des revendications précédentes comme réactif de détection de la MRM de la LLC.

13. Kit de diagnostic pour la détection cytométrique en flux de la maladie résiduelle minimale (MRM) dans la leucémie lymphoïde chronique (LLC), comprenant au moins une composition de réactifs selon les revendications 1 à 11, éventuellement avec des instructions d'utilisation, un tampon et/ou des échantillons témoins.

14. Procédé cytométrique de détection de la maladie résiduelle minimale (MRM) dans la leucémie lymphoïde chronique (LLC), comprenant les étapes consistant à :
(i) mettre une aliquote d'un échantillon biologique comprenant des leucocytes en contact avec une composition de réactifs selon l'une quelconque des revendications 1 à 11,
(ii) analyser les leucocytes dans ladite aliquote dans un cytomètre en flux ou un cytomètre de masse ;
(iii) réaliser un portillonnage sur les cellules pour l'expression des marqueurs choisis détectés par les anticorps conjugués présents dans la composition de réactifs ; et
(iv) distinguer les cellules normales des cellules malignes, sur la base du profil d'expression des marqueurs multiples, de préférence dans laquelle l'étape (iv) comprend la discrimination entre des lymphocytes B CD5+ CD27-/+ CD180hi normaux/réactifs et des cellules de LLC ou des cellules MBL de type LLC CD5+ CD27+ CD180-/low.

15. Procédé selon la revendication 14, dans laquelle l'échantillon est choisi dans le groupe constitué par le sang (frais ou stabilisé), la moelle osseuse (MO), le liquide céphalorachidien, la salive, les larmes, les épanchements pleuraux, le liquide péritonéal, le lavage bronchoalvéolaire, le thymus, les ganglions lymphatiques, les masses médiastinales et abdominales, et le prélèvement de rate, de préférence dans laquelle l'échantillon est un échantillon de sang ou de moelle osseuse.

16. Procédé selon la revendication 14 ou la revendication 15, dans laquelle l'étape (iv) implique une analyse multivariée, de préférence une analyse en composantes principales (ACP) et/ou une analyse canonique.
